(19) Europäisches Patentamt / European Patent Office / Office européen des brevets

(11) **EP 3 936 926 A1**

(12) **EUROPEAN PATENT APPLICATION**

(43) Date of publication:
**12.01.2022 Bulletin 2022/02**

(51) Int Cl.:
***G02C 7/02*** *(2006.01)*  ***G02C 7/04*** *(2006.01)*

(21) Application number: **21183719.0**

(22) Date of filing: **05.07.2021**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.07.2020 US 202063049425 P**
**17.03.2021 US 202117204692**

(71) Applicant: **Ovitz Corporation Rochester, NY 14604 (US)**

(72) Inventor: **Brown, Nicolas Scott Rochester, NY 14604 (US)**

(74) Representative: **Ostertag & Partner Patentanwälte mbB**
**Azenbergstraße 35**
**70174 Stuttgart (DE)**

(54) **LENS WITH MARKING PATTERN FOR CHARACTERIZING HIGH-ORDER ABERRATIONS**

(57) A contact lens with a plurality of marks for indicating a position of the contact lens and a rotation of the contact lens while the contact lens is positioned on an eye of a user is described. The plurality of marks includes at least one mark that is scribed on the contact lens. A method for making the contact lens and an optical device for use with the contact lens are also described.

Figure 1A

EP 3 936 926 A1

## Description

TECHNICAL FIELD

This relates generally to methods for determining a position of a corrective lens on an eye for compensation of higher-order aberrations, and contact lenses used for determining their positions on eyes.

BACKGROUND

Eyes are important organs, which play a critical role in human's visual perception. An eye has a roughly spherical shape and includes multiple elements, such as cornea, lens, vitreous humour, and retina. Imperfections in these components can cause reduction or loss of vision. For example, too much or too little optical power in the eye can lead to blurring of the vision (e.g., near-sightedness or far-sightedness), and astigmatism can also cause blurring of the vision.

Corrective lenses (e.g., glasses and contact lenses) are frequently used to compensate for blurring caused by too much or too little optical power and/or astigmatism. However, when eyes have higher-order aberrations (e.g., aberrations higher than astigmatism in the Zernike polynomial model of aberrations), conventional corrective lenses have not been effective at compensating for all of the aberrations associated with the eyes, resulting in blurry images even when corrective lenses are used.

SUMMARY

Accordingly, there is a need for corrective lenses that can compensate for higher-order aberrations. However, there is a variation in the structure and orientation of an eye between patients (and even different eyes of a same patient), and thus, a contact lens placed on an eye will settle in different positions and orientations for different patients (or different eyes). Proper alignment of the corrective lens to the patient's eye is required in order to provide an accurate correction or compensation of the higher-order aberrations in the eye. Thus, the position (e.g., lateral displacements and orientation) information for a contact lens is required along with vision information for effective correction or compensation of the higher-order aberrations in the eye, and devices and methods that can provide the position information along with the vision information are needed.

The above deficiencies and other problems associated with conventional devices and methods are reduced or eliminated by lenses, devices, and methods described herein. As described herein, a reference lens with markings (also called herein a predicate lens) may be used to facilitate collection of the position information.

In accordance with some embodiments, a contact lens includes an optically transparent lens with a plurality of marks for indicating a position of the contact lens and a rotation of the contact lens while the contact lens is positioned on an eye of a user.

In some embodiments, the plurality of marks includes a first set of one or more marks for indicating the position of the contact lens.

In some embodiments, the plurality of marks includes a second set of one or more marks for indicating the rotation of the contact lens.

In some embodiments, the rotation of the contact lens includes rotation about an optical axis of the contact lens and rotation about an axis orthogonal to the optical axis of the contact lens.

In some embodiments, the first set of one or more marks includes a mark positioned at a center of the contact lens.

In some embodiments, the first set of one or more marks includes one or more marks positioned at a first distance from a center of the contact lens.

In some embodiments, the second set of one or more marks includes one or more marks positioned at a second distance, distinct from the first distance, from the center of the contact lens.

In some embodiments, the first set of one or more marks and the second set of one or more marks are mutually exclusive.

In some embodiments, the first set of one or more marks and the second set of one or more marks have at least one common mark.

In some embodiments, the first set of one or more marks includes at least one mark that is not included in the second set of one or more marks or the second set of one or more marks includes at least one mark that is not included in the first set of one or more marks.

In some embodiments, the plurality of marks also includes a third set of one or more marks for indicating a tilt of the contact lens while the contact lens is positioned on the eye of the user.

In some embodiments, the third set of one or more marks includes one or more marks positioned at a third distance, distinct from the first distance, from a center of the contact lens.

In some embodiments, the third distance is different from the second distance.

In some embodiments, the third distance is identical to the second distance.

In some embodiments, the contact lens includes one or more quadrants without any of the plurality of marks.

In some embodiments, the contact lens includes one or more quadrants without any of the plurality of marks.

In some embodiments, each mark of the plurality of marks has a circularly symmetric shape.

In some embodiments, at least one of the plurality of marks has a shape that is not circularly symmetric.

In accordance with some embodiments, a contact lens includes an optically transparent lens with a plurality of marks, the plurality of marks including a first set of one or more marks including one or more marks positioned at a first distance from a center of the contact lens, and a second set of one or more marks including one or more marks positioned at a second distance, distinct from the first distance, from the center of the contact lens.

In some embodiments, the lens has a lens center identifiable according to one or more marks of the plurality of marks.

In some embodiments, one or more marks of the plurality of marks include a fluorescent material.

In accordance with some embodiments, a method includes obtaining, with an optical device, information indicating at least one of: a position and a rotation of any contact lens described herein on an eye wearing the contact lens.

In accordance with some embodiments, a contact lens with a plurality of marks for indicating a position of the contact lens and a rotation of the contact lens while the contact lens is positioned on an eye of a user is described. The plurality of marks includes at least one mark that is scribed on the contact lens.

In accordance with some embodiments, a method of making a contact lens includes obtaining a first contact lens; and forming one or more marks on a surface of the first contact lens for indicating a position of the contact lens and a rotation of the contact lens. In some embodiments, the one or more marks include one or more indentations. In some embodiments, the one or more marks are one or more indentations.

In accordance with some embodiments, a device includes a light source for providing light to an eye so that at least a portion of the light is returned to the device; and a first image sensor positioned to image a pupil region of the eye for capturing a shadow of one or more marks on a contact lens positioned on the eye.

In accordance with some embodiments, a method includes providing light to an eye wearing a contact lens with one or more marks; receiving at least a portion of the light returned from the eye; and imaging the received light to form an image with respective shadows of the one or more marks.

Thus, the disclosed embodiments provide contact lenses and methods of collecting position information for contact lenses, which can be used to accurately determine a position of a position reference point (e.g., a visual axis) of an eye relative to a contact lens (or vice versa), in conjunction with vision information. Such information, in turn, allows design and manufacturing of customized (e.g., personalized) contact lenses that can compensate for higher-order aberrations in a particular eye.

BRIEF DESCRIPTION OF THE DRAWINGS

For a better understanding of the various described embodiments, reference should be made to the Description of Embodiments below, in conjunction with the following drawings in which like reference numerals refer to corresponding parts throughout the figures.

Figure 1A is a schematic diagram showing a system for vision characterization in accordance with some embodiments.

Figures 1B and 1C illustrate optical components of an optical device in accordance with some embodiments.

Figure 1D illustrates wavefront sensing with the optical device shown in Figures 1B and 1C, in accordance with some embodiments.

Figure 1E illustrates imaging with the optical device shown in Figures 1B and 1C, in accordance with some embodiments.

Figures 1F and 1G illustrate optical components of an optical device in accordance with some other embodiments.

Figure 1H is a front view of a measurement instrument in accordance with some embodiments.

Figure 2 is a block diagram illustrating electronic components of an optical device in accordance with some embodiments.

Figures 3A-3D are schematic diagrams illustrating correction of higher-order aberrations in accordance with some embodiments.

Figure 3E shows an image of a reference lens with marks in accordance with some embodiments.

Figure 4A is a schematic diagram illustrating a perspective view of an eye and aspects of lens positioning that relate to design and fitting of the scleral contact lens.

Figure 4B is a schematic diagram illustrating a plan view of the eye and the lens shown in Figure 4A, taken along the visual axis.

Figure 5A is a schematic diagram illustrating a reference lens with marks in accordance with some embodiments.

Figure 5B is a schematic diagram illustrating partial obstruction of the marks shown in Figure 5A.

Figure 6 is a schematic diagram illustrating a reference lens with a different marking pattern in accordance with some embodiments.

Figures 7A and 7B are schematic diagrams illustrating reference lenses with a reduced number of marks in accordance with some embodiments.

Figures 8A and 8B are front elevational views of a reference lens with marks arranged in a concentric pattern, in accordance with some embodiments.

Figures 8C and 8D are front elevational views of another reference lens with marks arranged in a concentric pattern, in accordance with some embodiments.

Figure 9 illustrates examples of tilting of the lens.

Figure 10 is a schematic diagram illustrating a side view of lens and a method for determining a tilt of the lens in accordance with some embodiments.

Figure 11 is a front view showing exemplary lens positioning on an eye.

Figure 12A is a schematic diagram illustrating a reference lens with marks in accordance with some embodiments.

Figure 12B shows a lens with marks, positioned on an eye, in accordance with some embodiments.

Figure 12C shows a lens with highlighted marks, positioned on an eye, in accordance with some embodiments.

Figures 13A and 13B are schematic diagrams illustrating methods of making a contact lens with one or more indentations in accordance with some embodiments.

Figures 14A and 14B illustrate wavefront sensing and mark imaging with an optical device in accordance with some embodiments.

Figures 14C and 14D illustrate another optical device capable of wavefront sensing and mark imaging in accordance with some embodiments.

Figure 15 is an image of a contact lens with indentation marks in accordance with some embodiments.

These figures are not drawn to scale unless indicated otherwise.

DETAILED DESCRIPTION

Reference will be made to embodiments, examples of which are illustrated in the accompanying drawings. In the following description, numerous specific details are set forth in order to provide a thorough understanding of the various described embodiments. However, it will be apparent to one of ordinary skill in the art that the various described embodiments may be practiced without these particular details. In other instances, methods, procedures, components, circuits, and networks that are well-known to those of ordinary skill in the art are not described in detail so as not to unnecessarily obscure aspects of the embodiments.

It will also be understood that, although the terms first, second, etc. may be used herein to describe various elements, these elements should not be limited by these terms. These terms are only used to distinguish one element from another. For example, a first image sensor could be termed a second image sensor, and, similarly, a second image sensor could be termed a first image sensor, without departing from the scope of the various described embodiments. The first image sensor and the second image sensor are both image sensors, but they are not the same image sensor.

The terminology used in the description of the embodiments herein is for the purpose of describing particular embodiments only and is not intended to be limiting of the invention. As used in the description of the invention and the appended claims, the singular forms "a," "an," and "the" are intended to include the plural forms as well, unless the context clearly indicates otherwise. It will also be understood that the term "and/or" as used herein refers to and encompasses any and all possible combinations of one or more of the associated listed items. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

As used herein, the term "if' may be construed to mean "when" or "upon" or "in response to determining" or "in response to detecting," depending on the context. Similarly, the phrase "if it is determined" or "if [a stated condition or event] is detected" may be construed to mean "upon determining" or "in response to determining" or "upon detecting (the stated condition or event)" or "in response to detecting (the stated condition or event)," depending on the context.

A corrective lens (e.g., contact lens) designed to compensate for higher-order aberrations of an eye needs accurate positioning on an eye. If a corrective lens designed to compensate for higher-order aberrations of an eye is not placed accurately, the corrective lens may not be effective in compensating for higher-order aberrations of the eye and may even exacerbate the higher-order aberrations.

One of the additional challenges is that when a corrective lens (e.g., contact lens) is used to compensate for higher-order aberrations of an eye, an apex of a corrective lens is not necessarily positioned on a visual axis of the eye. Thus, a relative position between the visual axis of the eye and the apex of the corrective lens needs to be reflected in the design of the corrective lens. This requires accurate measurements of the visual axis of the eye and a position of the corrective lens on the eye. However, because the eye has a curved three-dimensional surface, conventional methods for determining the position of the corrective lens relative to the visual axis of the eye often have errors. Such errors hamper the performance of a corrective lens designed to compensate for higher-order aberrations. Thus, for designing a corrective lens that can compensate for the higher-order aberrations, an accurate measurement of the visual axis (or any other position reference point) of the eye may be necessary in some cases.

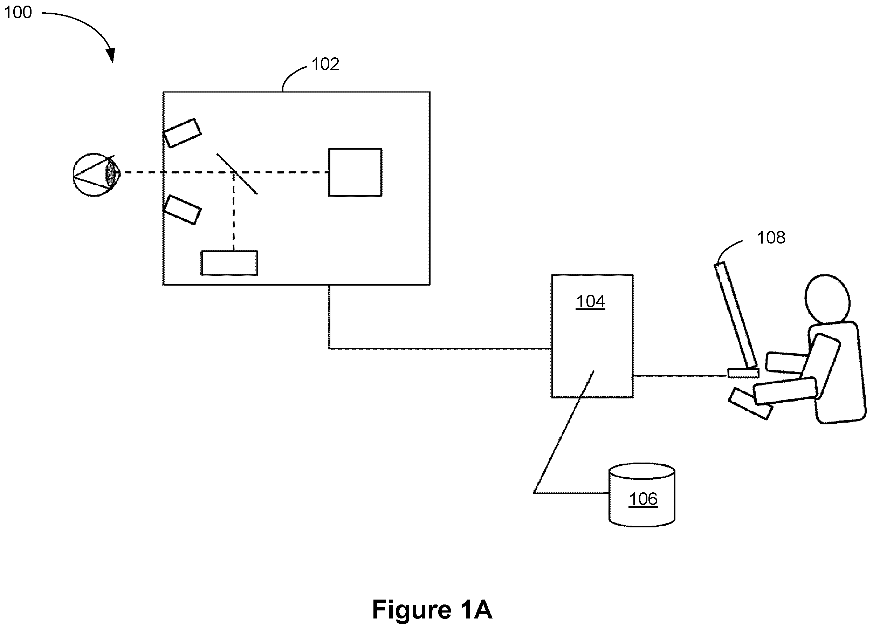

Figure 1A is a schematic diagram showing a system 100 for vision characterization in accordance with some embodiments. The system 100 includes a measurement device 102, a computer system 104, a database 106, and a display device 108. The measurement device 102 performs a vision characterization of an eye of a patient and provides imaging results and vision profile metrics of the characterized eye. The measurement device 102 includes a wavefront measurement device, such as a Shack-Hartmann wavefront sensor, that is configured to perform wavefront measurements. The display device 108 shows the imaging results and vision profile metrics acquired by the measurement device 102. In some cases, the display device 108 may provide a user (e.g., operator, optometrist, viewer, or practitioner) with one or more options or prompts to correct, validate, or confirm displayed results. The database 106 stores imaging results and vision profile metrics acquired by the measurement device 102 as well as any verified information provided by the user of the system 100. In response to receiving the results from the measurement device 102 and validation of displayed results from the user, the system 100 may generate a correction lens (e.g., contact lens) fabrication file for the patient that is stored in the database 106.

The computer system 104 may include one or more computers or central processing units (CPUs). The computer system 104 is in communication with each of the measurement device 102, the database 106, and the display device 108.

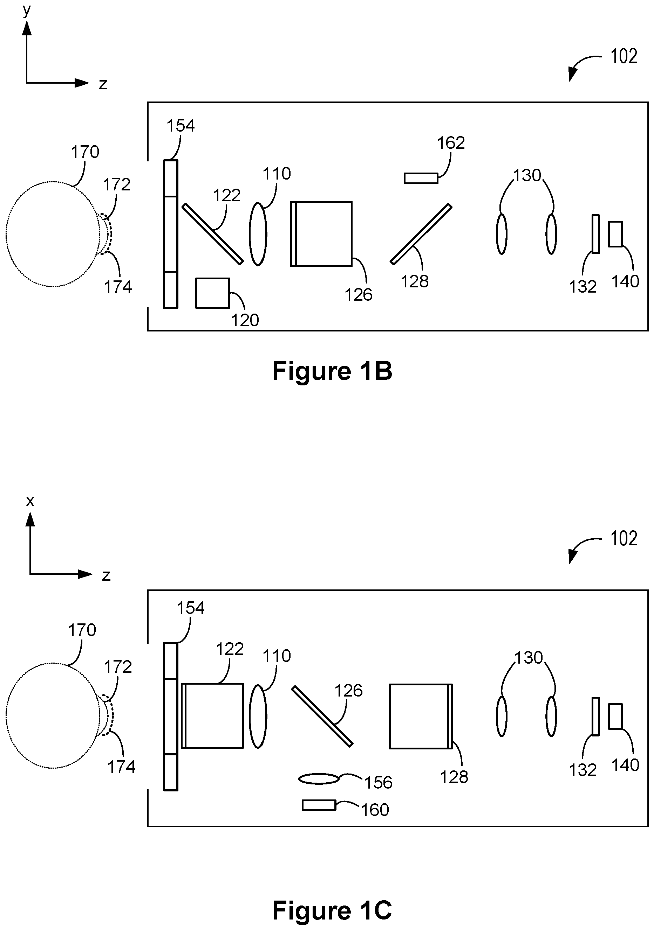

Figures 1B - 1E illustrate optical components of the measurement device 102 in accordance with some embodiments. Figure 1B shows a side view (e.g., a side elevational view) of the optical components of the measurement device 102, and Figure 1C is a top view (e.g., a plan view) of the optical components of the measurement device 102. One or more lenses 156 and second image sensor 160 shown in Figure 1C are not shown in Figure 1B to avoid obscuring other components of the measurement device 102 shown in Figure 1B. In Figure 1C, pattern 162 is not shown to avoid obscuring other components of the measurement device 102 shown in Figure 1C.

The measurement device 102 includes lens assembly 110. In some embodiments, lens assembly 110 includes one or more lenses. In some embodiments, lens assembly 110 is a doublet lens. For example, a doublet lens is selected to reduce spherical aberration and other aberrations (e.g., coma and/or chromatic aberration). In some embodiments, lens assembly 110 is a triplet lens. In some embodiments, lens assembly 110 is a singlet lens. In some embodiments, lens assembly 110 includes two or more separate lenses. In some embodiments, lens assembly 110 includes an aspheric lens. In some embodiments, a working distance of lens assembly 110 is between 10-100 mm (e.g., between 10-90 mm, 10-80 mm, 10-70 mm, 10-60 mm, 10-50 mm, 15-90 mm, 15-80 mm, 15-70 mm, 15-60 mm, 15-50 mm, 20-90 mm, 20-80 mm, 20-70 mm, 20-60 mm, 20-50 mm, 25-90 mm, 25-80 mm, 25-70 mm, 25-60 mm, or 25-50 mm). In some embodiments, when the lens assembly includes two or more lenses, an effective focal length of a first lens (e.g., the lens positioned closest to the pupil plane) is between 10-150 mm (e.g., between 10-140 mm, 10-130 mm, 10-120 mm, 10-110 mm, 10-100 mm, 10-90 mm, 10-80 mm, 10-70 mm, 10-60 mm, 10-50 mm, 15-150 mm, 15-130 mm, 15-120 mm, 15-110 mm, 15-100 mm, 15-90 mm, 15-80 mm, 15-70 mm, 15-60 mm, 15-50 mm, 20-150 mm, 20-130 mm, 20-120 mm, 20-110 mm, 20-100 mm, 20-90 mm, 20-80 mm, 20-70 mm, 20-60 mm, 20-50 mm, 25-150 mm, 25-130 mm, 25-120 mm, 25-110 mm, 25-100 mm, 25-90 mm, 25-80 mm, 25-70 mm, 25-60 mm, 25-50 mm, 30-150 mm, 30-130 mm, 30-120 mm, 30-110 mm, 30-100 mm, 30-90 mm, 30-80 mm, 30-70 mm, 30-60 mm, 30-50 mm, 35-150 mm, 35-130 mm, 35-120 mm, 35-110 mm, 35-100 mm, 35-90 mm, 35-80 mm, 35-70 mm, 35-60 mm, 35-50 mm, 40-150 mm, 40-130 mm, 40-120 mm, 40-110 mm, 40-100 mm, 40-90 mm, 40-80 mm, 40-70 mm, 40-60 mm, 40-50 mm, 45-150 mm, 45-130 mm, 45-120 mm, 45-110 mm, 45-100 mm, 45-90 mm, 45-80 mm, 45-70 mm, 45-60 mm, 45-50 mm, 50-150 mm, 50-130 mm, 50-120 mm, 50-110 mm, 50-100 mm, 50-90 mm, 50-80 mm, 50-70 mm, or 50-60 mm). In some embodiments, for an 8 mm pupil diameter, the lens diameter is 16-24 mm. In some embodiments, for a 7 mm pupil diameter, the lens diameter is 12-20 mm. In some embodiments, the f-number of lens assembly is between 2 and 5. The use of a common lens assembly (e.g., lens assembly 110) in both a wavefront sensor and a contact lens center sensor allows the integration of the wavefront sensor and the contact lens center sensor without needing large diameter optics.

The measurement device 102 also includes a wavefront sensor. In some embodiments, the wavefront sensor includes first light source 120, lens assembly 110, an array of lenses 132 (also called herein lenslets), and first image sensor 140. In some embodiments, the wavefront sensor includes additional components (e.g., one or more lenses 130). In some embodiments, the wavefront sensor does not include such additional components.

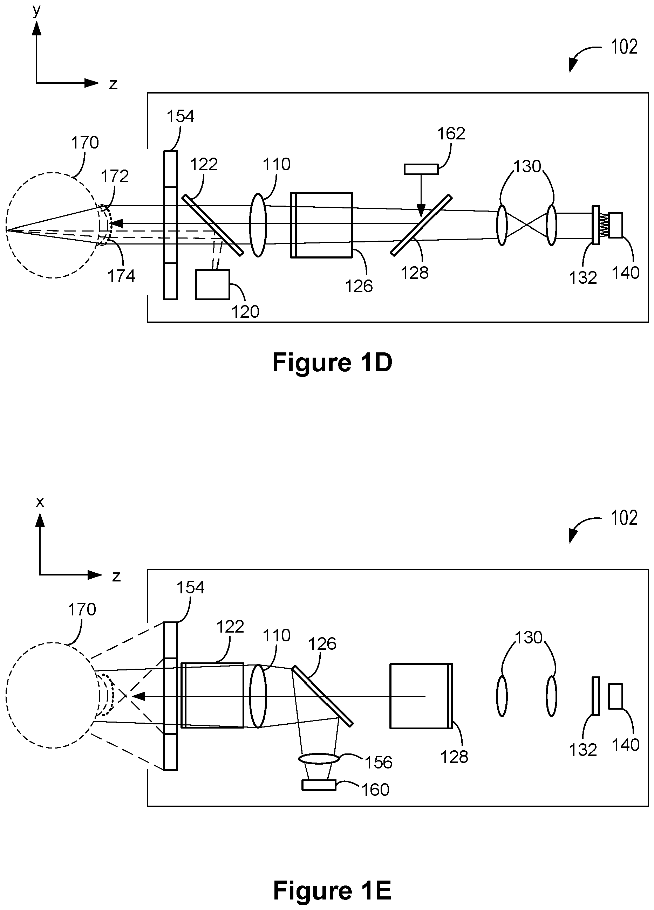

First light source 120 is configured to emit first light and transfer the first light emitted from the first light source toward eye 170, as depicted in Figure 1D.

Figures 1B - 1E include eye 170, its components (e.g., cornea 172), and contact lens 174 to illustrate the operations of the measurement device 102 with eye 170 and contact lens 174. By performing measurements on eye 170 with contact lens 174, aberrations in eye 170 as modified by contact lens 174 may be detected. In addition, the position of contact lens 174 relative to eye 170 may be detected. However, eye 170, its components, and contact lens 174 are not part of the measurement device 102.

Turning back to Figure 1B, in some embodiments, first light source 120 is configured to emit light of a single wavelength or a narrow band of wavelengths. Exemplary first light source 120 includes a laser (e.g., a laser diode) or a light-emitting diode (LED).

In some embodiments, first light source 120 includes one or more lenses to change the divergence of the light emitted from first light source 120 so that the light, after passing through the one or more lenses, is collimated.

In some embodiments, first light source 120 includes a pinhole (e.g., having a diameter of 1 mm or less, such as 400 $\mu$m, 500 $\mu$m, 600 $\mu$m, 700 $\mu$m, 800 $\mu$m, 900 $\mu$m, and 1 mm).

In some cases, an anti-reflection coating is applied on a back surface (and optionally, a front surface) of lens assembly 110 to reduce reflection. In some embodiments, first light source 120 is configured to transfer the first light emitted from first light source 120 off an optical axis of the measurement device 102 (e.g., an optical axis of lens assembly 110), as shown in Figure 1D (e.g., the first light emitted from first light source 120 propagates parallel to, and offset from, the optical axis of lens assembly 110). This reduces back reflection of the first light emitted from first light source 120, by cornea 172, toward first image sensor 140. In some embodiments, the wavefront sensor includes a quarter-wave plate to reduce back reflection, of the first light, from lens assembly 110 (e.g., light reflected from lens assembly 110 is attenuated by the quarter-wave plate). In some embodiments, the quarter-wave plate is located between beam steerer 122 and first image sensor 140.

First image sensor 140 is configured to receive light, from eye 170, transmitted through lens assembly 110 and the array of lenses 132. In some embodiments, the light from eye 170 includes light scattered at a retina or fovea of eye 170 (in response to the first light from first light source 120). For example, as shown in Figure ID, light from eye 170 passes multiple optical elements, such as beam steerer 122, lens assembly 110, beam steerer 126, beam steerer 128, and lenses 130, and reaches first image sensor 140.

Beam steerer 122 is configured to reflect light from light source 120 and transmit light from eye 170, as shown in Figure 1D. Alternatively, beam steerer 122 is configured to transmit light from light source 120 and reflect light from eye 170. In some embodiments, beam steerer 122 is a beam splitter (e.g., 50:50 beam splitter, polarizing beam splitter, etc.). In some embodiments, beam steerer 122 is a wedge prism, and when first light source 120 is configured to have a linear polarization, the polarization of the light emitted from first light source 120 is configured to reflect at least partly by the wedge prism. Light of a polarization that is orthogonal to the linear polarization of the light emitted from first light source 120 is transmitted through the wedge prism. In some cases, the wedge prism also reduces light reflected from cornea 172 of eye 170.

In some embodiments, beam steerer 122 is tilted at such an angle (e.g., an angle between the optical axis of the measurement device 102 and a surface normal of beam steerer 122 is at an angle less than 45°, such as 30°) so that the space occupied by beam steerer 122 is reduced.

In some embodiments, the measurement device 102 includes one or more lenses 130 to modify a working distance of the measurement device 102.

The array of lenses 132 is arranged to focus incoming light onto multiple spots, which are imaged by first image sensor 140. As in Shack-Hartmann wavefront sensor, an aberration in a wavefront causes displacements (or disappearances) of the spots on first image sensor 140. In some embodiments, a Hartmann array is used instead of the array of lenses 132. A Hartmann array is a plate with an array of apertures (e.g., through-holes) defined therein.

In some embodiments, one or more lenses 130 and the array of lenses 132 are arranged such that the wavefront sensor is configured to measure a reduced range of optical power. A wavefront sensor that is capable of measuring a wide range of optical power may have less accuracy than a wavefront sensor that is capable of measuring a narrow range of optical power. Thus, when a high accuracy in wavefront sensor measurements is desired, the wavefront sensor can be designed to cover a narrow range of optical power. For example, a wavefront sensor for diagnosing low and medium myopia can be configured with a narrow range of optical power between 0 and -6.0 diopters, with its range centering around -3.0 diopters. Although such a wavefront sensor may not provide accurate measurements for diagnosing hyperopia (or determining a prescription for hyperopia), the wavefront sensor would provide more accurate measurements for diagnosing myopia (or determining a prescription for myopia) than a wavefront sensor that can cover both hyperopia and myopia (e.g., from -6.0 to +6.0 diopters). In addition, there are certain populations in which it is preferable to maintain a center of the range at a non-zero value. For example, in some Asian populations, the optical power may range from +6.0 to -14.0 diopters (with the center of the range at -4.0 diopters), whereas in some Caucasian populations, the optical power may range from +8.0 to -12.0 diopters (with the center of the range at -2.0 diopters). The center of the range can be shifted by moving the lenses (e.g., one or more lenses 130 and/or the array of lenses 132). For example, defocusing light from eye 170 can shift the center of the range.

The measurement device 102 further includes a contact lens center sensor (or a corneal vertex sensor). In some embodiments, the contact lens center sensor includes lens assembly 110, second light source 154, and second image sensor 160. In some embodiments, as shown in Figure 1C, second image sensor 160 is distinct from first image sensor 140. In some embodiments, the wavefront sensor includes additional components that are not included in the contact lens center sensor (e.g., array of lenses 132).

Second light source 154 is configured to emit second light and transfer the second light emitted from second light source 154 toward eye 170. As shown in Figure IE, in some embodiments, second light source 154 is configured to transfer the second light emitted from second light source 154 toward eye 170 without transmitting the second light emitted from second light source 154 through lens assembly 110 (e.g., second light from second light source 154 is directly transferred to eye 170 without passing through lens assembly 110).

In some embodiments, the measurement device 102 includes beam steerer 126 configured to transfer light from eye 170, transmitted through lens assembly 110, toward first image sensor 140 and/or second image sensor 160. For example, when the measurement device 102 is configured for wavefront sensing (e.g., when light from first light source 120 is transferred toward eye 170), beam steerer 126 transmits light from eye 170 toward first image sensor 140, and when the measurement device 102 is configured for contact lens center determination (e.g., when light from second light source 154 is transferred toward eye 170), beam steerer 126 transmits light from eye 170 toward second image sensor 160.

Second light source 154 is distinct from first light source 120. In some embodiments, first light source 120 and second light source 154 emit light of different wavelengths (e.g., first light source 120 emits light of 900 nm wavelength, and second light source 154 emits light of 800 nm wavelength; alternatively, first light source 120 emits light of 850 nm wavelength, and second light source 154 emits light of 950 nm wavelength).

In some embodiments, beam steerer 126 is a dichroic mirror (e.g., a mirror that is configured to transmit the first light from first light source 120 and reflect the second light from second light source 154, or alternatively, reflect the first light from first light source 120 and transmit the second light from second light source 154). In some embodiments, beam steerer 126 is a movable mirror (e.g., a mirror that can flip or rotate to steer light toward first image sensor 140 and second image sensor 160). In some embodiments, beam steerer 126 is a beam splitter. In some embodiments, beam steerer 126 is configured to transmit light of a first polarization and reflect light of a second polarization that is distinct from (e.g., orthogonal to) the first polarization. In some embodiments, beam steerer 126 is configured to reflect light of the first polarization and transmit light of the second polarization.

In some embodiments, second light source 154 is configured to project a predefined pattern of light on the eye. In some embodiments, second light source 154 is configured to project an array of spots on the eye. In some embodiments, the array of spots is arranged in a grid pattern.

In some embodiments, second light source 154 includes one or more light emitters (e.g., light-emitting diodes) and diffuser (e.g., a diffuser plate having an array of spots).

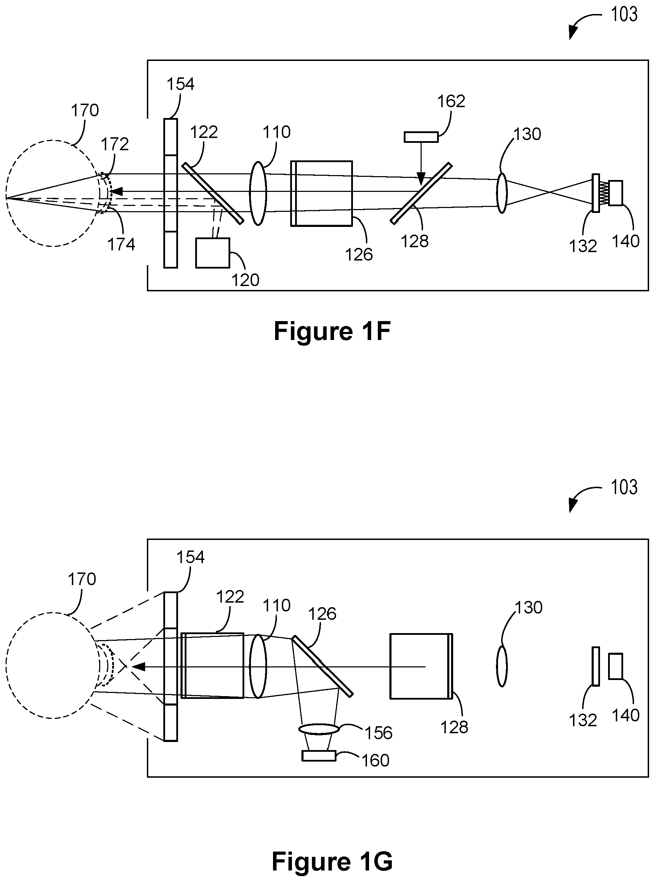

Figures 1F and 1G illustrate optical components of a measurement instrument 103 in accordance with some other embodiments. Measurement instrument 103 is similar to the measurement device 102 shown in Figures 1B - 1E except that measurement instrument 103 includes only one lens 130.

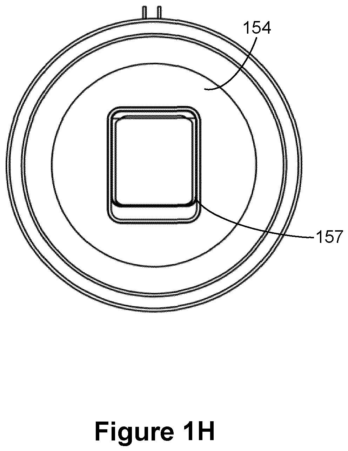

Figure 1H is a front view of the measurement device 102 in accordance with some embodiments. The side view of the measurement device 102 shown in Figure 1H corresponds to a view of the measurement device 102 seen from a side that is adjacent to second light source 154. In Figure 1H, the measurement device 102 includes second light source 154, which has a circular shape with a rectangular hole 157 defined in it. Second light source 154 shown in Figure 1H projects a pattern of light.

Turning back to Figure IE, second image sensor 160 is configured to receive light, from eye 170. In some embodiments, the light from eye 170 includes light reflected from cornea 172 of eye 170 (in response to the second light from second light source 154). For example, as shown in Figure IE, light from eye 170 (e.g., light reflected from cornea 172) interacts with multiple optical elements, such as lens assembly 110, beam steerer 122, lens 124, beam steerer 126, and one or more lenses 156, and reaches second image sensor 160.

In some embodiments, the lenses in the contact lens center sensor (e.g., lens assembly 110 and one or more lenses 156) are configured to image a pattern of light projected on cornea 172 onto second image sensor 160.

In some embodiments, second image sensor 160 collects an image of a combination of eye 170 and contact lens 174. From the image, the position and orientation of contact lens 174 relative to eye 170 (e.g., relative to a pupil center or a visual axis of eye 170) may be determined, as described herein.

In some embodiments, the measurement device 102 includes pattern 162 and beam steerer 128. Pattern 162 is an image that is projected toward eye 170 to facilitate positioning of eye 170. In some embodiments, pattern 162 includes an image of an object (e.g., balloon), an abstract shape (e.g., a cross), or a pattern of light (e.g., a shape having a blurry edge).

In some embodiments, beam steerer 128 is a dichroic mirror (e.g., a mirror that is configured to transmit the light from eye 170 and reflect light from pattern 162, or alternatively, reflect light from eye 170 and transmit light from pattern 162). In some embodiments, beam steerer 128 is a movable mirror. In some embodiments, beam steerer 128 is a beam splitter. In some embodiments, beam steerer 128 is configured to transmit light of a first polarization and reflect light of a second polarization that is distinct from (e.g., orthogonal to) the first polarization. In some embodiments, beam steerer 128 is configured to reflect light of the first polarization and transmit light of the second polarization.

Figure 1D illustrates operation of the measurement device 102 for wavefront sensing without operations for determining a contact lens center and Figure 1E illustrates operation of the measurement device 102 for determining a contact lens center without wavefront sensing. In some embodiments, the measurement device 102 sequentially operates between wavefront sensing and determining a contact lens center. For example, in some cases, the measurement device 102 performs wavefront sensing and subsequently, determines a contact lens center. In some other cases, the measurement device 102 determines a contact lens center, and subsequently performs wavefront sensing. In some embodiments, the measurement device 102 switches between wavefront sensing and determining a contact lens center. In some embodiments, the measurement device 102 repeats wavefront sensing and determining a contact lens center. In some embodiments, the measurement device 102 operates for wavefront sensing concurrently with determining a contact lens center (e.g., light from first light source 120 and light from second light source 154 are delivered toward eye 170 at the same time, and first image sensor 140 and second image sensor 160 collect images at the same time). For brevity, such details are not repeated herein.

In some embodiments, light from pattern 162 is projected toward eye 170 while the measurement device 102 operates for wavefront sensing (as shown in Figure ID). In some embodiments, light from pattern 162 is projected toward eye 170 while device operates for determining a contact lens center (as shown in Figure IE).

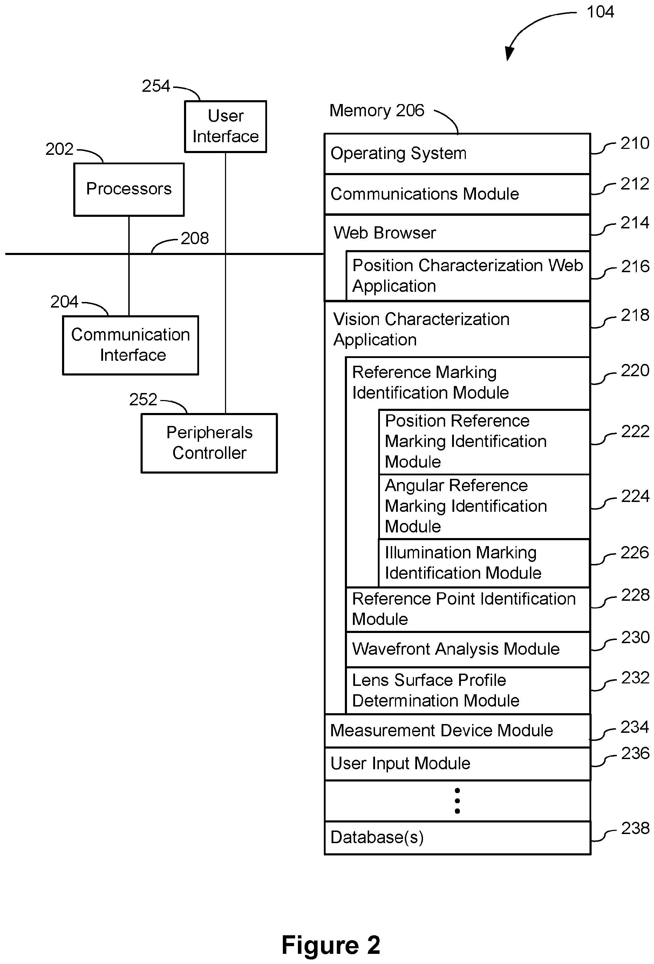

Figure 2 shows block diagram illustrating electronic components of computer system 104 in accordance with some embodiments. Computer system 104 includes one or more processing units 202 (central processing units, application processing units, application-specific integrated circuit, etc., which are also called herein processors), one or more network or other communications interfaces 204, memory 206, and one or more communication buses 208 for interconnecting these components. In some embodiments, communication buses 208 include circuitry (sometimes called a chipset) that interconnects and controls communications between system components. In some embodiments, system 100 includes a user interface 254 (e.g., a user interface having the display device 108, which can be used for displaying acquired images, one or more buttons, and/or other input devices). In some embodiments, computer system 104 also includes peripherals controller 252, which is configured to control operations of components of the measurement device 102, such as first light source 120, first image sensor 140, second light source 150, and second image sensor 160 (e.g., initiating respective light sources to emit light, and/or receiving information, such as images, from respective image sensors).

In some embodiments, communications interfaces 204 include wired communications interfaces and/or wireless communications interfaces (e.g., Wi-Fi, Bluetooth, etc.).

Memory 206 of computer system 104 includes high-speed random access memory, such as DRAM, SRAM, DDR RAM or other random access solid state memory devices; and may include non-volatile memory, such as one or more magnetic disk storage devices, optical disk storage devices, flash memory devices, or other non-volatile solid state storage devices. Memory 206 may optionally include one or more storage devices remotely located from the processors 202. Memory 206, or alternately the non-volatile memory device(s) within memory 206, comprises a computer readable storage medium (which includes a non-transitory computer readable storage medium and/or a transitory computer readable storage medium). In some embodiments, memory 206 includes a removable storage device (e.g., Secure Digital memory card, Universal Serial Bus memory device, etc.). In some embodiments, memory 206 or the computer readable storage medium of memory 206 stores the following programs, modules and data structures, or a subset thereof:

- operating system 210 that includes procedures for handling various basic system services and for performing hardware dependent tasks;
- network communication module (or instructions) 212 that is used for connecting computer system 104 to other computers (e.g., clients and/or servers) via one or more communications interfaces 204 and one or more communications networks, such as the Internet, other wide area networks, local area networks, metropolitan area networks, and so on;
- vision characterization application 218, or position characterization web application 216 that runs in a web browser 214, that characterizes position information from an image of an eye and markings;
- measurement device module 234 that controls operations of the light sources and the image sensors in the measurement device 102 (e.g., for receiving images from the measurement device 102);
- user input module 236 configured for handling user inputs on computer system 104 (e.g., pressing of buttons on computer system 104 or pressing of buttons on a user interface, such as a keyboard, mouse, or touch-sensitive display, that is in communication with computer system 104); and

- one or more databases 238 (e.g., database 106) that store information acquired by the measurement device 102.

In some embodiments, memory 206 also includes one or both of:

- user information (e.g., information necessary for authenticating a user of computer system 104); and
- patient information (e.g., optical measurement results and/or information that can identify patients whose optical measurement results are stored in the one or more databases 238 on computer system 104).

In some embodiments, vision characterization application 218, or vision characterization web application 216, includes the following programs, modules and data structures, or a subset or superset thereof:

- reference marking identification module 220 configured for identifying (e.g., automatically identifying) one or more reference markings in an image captured (e.g., recorded, acquired) by the measurement device 102, which may include one or more of the following:

  ◦ periphery reference marking identification module 222 configured for identifying (e.g., automatically identifying) one or more periphery reference markings in an image captured (e.g., recorded, acquired) by the measurement device 102;
  ◦ angular reference marking identification module 224 configured for identifying (e.g., automatically identifying) one or more angular reference markings in an image captured (e.g., recorded, acquired) by the measurement device 102; and
  ◦ illumination marking identification module 226 configured for identifying (e.g., automatically identifying) one or more illumination markings in an image captured (e.g., recorded, acquired) by the measurement device 102;

- reference point identification module 228 configured for identifying (e.g., automatically identifying) a position reference point of a patient's eye based on an image captured (e.g., recorded, acquired) by the measurement device 102;
- wavefront analysis module 230 configured for analyzing the wavefront measured for a patient's eye(s) using the measurement device 102; and
- lens surface profile determination module 232 configured for determining a lens surface profile for a patient's eye(s) based the wavefront measured for a patient's eye and the positions of reference markings.

In some embodiments, wavefront analysis module 230 includes the following programs and modules, or a subset or superset thereof:

- an analysis module configured for analyzing images received from first image sensor 140; and
- a first presentation module configured for presenting measurement and analysis results from first analysis module (e.g., graphically displaying images received from first image sensor 140, presenting aberrations shown in images received from first image sensor 140, sending the results to another computer, etc.).

In some embodiments, measurement device module 234 includes the following programs and modules, or a subset or superset thereof:

- a light source module configured for initiating first light source 120 (through peripherals controller 252) to emit light;
- an image sensing module configured for receiving images from first image sensor 140;
- a light source module configured for initiating second light source 154 (through peripherals controller 252) to emit light;
- an image sensing module configured for receiving images from second image sensor 160;
- an image acquisition module configured for capturing one or more images of a patient's eye(s) using the measurement device 102; and
- an image stabilization module configured for reducing blurring during acquisition of images by image sensors.

In some embodiments, the computer system 104 may include other modules such as:

- an analysis module configured for analyzing images received from second image sensor 160 (e.g., determining a center of a projected pattern of light);
- a presentation module configured for presenting measurement and analysis results from second analysis module (e.g., graphically displaying images received from second image sensor 160, presenting cornea curvatures determined from images received from second image sensor 160, sending the results to another computer, etc.);
- a spot array analysis module configured for analyzing spot arrays (e.g., measuring displacements and/or disappearances of spots in the spot arrays); and
- a centering module configured for determining a center of a projected pattern of light.

In some embodiments, a first image sensing module initiates execution of the image stabilization module to reduce blurring during acquisition of images by first image sensor 140, and a second image sensing module initiates execution of the image stabilization module to reduce blurring during acquisition of images by second image sensor 160.

In some embodiments, a first analysis module initiates execution of spot array analysis module to analyze spot arrays in images acquired by first image sensor 140, and a second analysis module initiates execution of spot array analysis module to analyze spot arrays in images acquired by second image sensor 160.

In some embodiments, a first analysis module initiates execution of spot array analysis module to analyze spot arrays in images acquired by first image sensor 140, and a second analysis module initiates execution of centering module to analyze images acquired by second image sensor 160.

In some embodiments, the one or more databases 238 may store any of: wavefront image data, including information representing the light received by the first image sensor (e.g., images received by the first image sensor), and pupil image data, including information representing the light received by the second image sensor (e.g., images received by the second image sensor).

Each of the above identified modules and applications correspond to a set of instructions for performing one or more functions described above. These modules (i.e., sets of instructions) need not be implemented as separate software programs, procedures or modules, and thus various subsets of these modules may be combined or otherwise re-arranged in various embodiments. In some embodiments, memory 206 may store a subset of the modules and data structures identified above. Furthermore, memory 206 may store additional modules and data structures not described above.

Notwithstanding the discrete blocks in Figure 2, these figures are intended to be a functional description of some embodiments, although, in some embodiments, the discrete blocks in Figure 2 can be a structural description of functional elements in the embodiments. One of ordinary skill in the art will recognize that an actual implementation might have the functional elements grouped or split among various components. In practice, and as recognized by those of ordinary skill in the art, items shown separately could be combined and some items could be separated. For example, in some embodiments, measurement device module 234 is part of vision characterization application 218 (or vision characterization web application 216). In other embodiments, reference marking identification module 220, wavefront analysis module 230, and lens surface profile determination module 232 are implemented as separate applications. In some embodiments, one or more programs, modules, or instructions may be implemented in measurement device 102 instead of computer system 104.

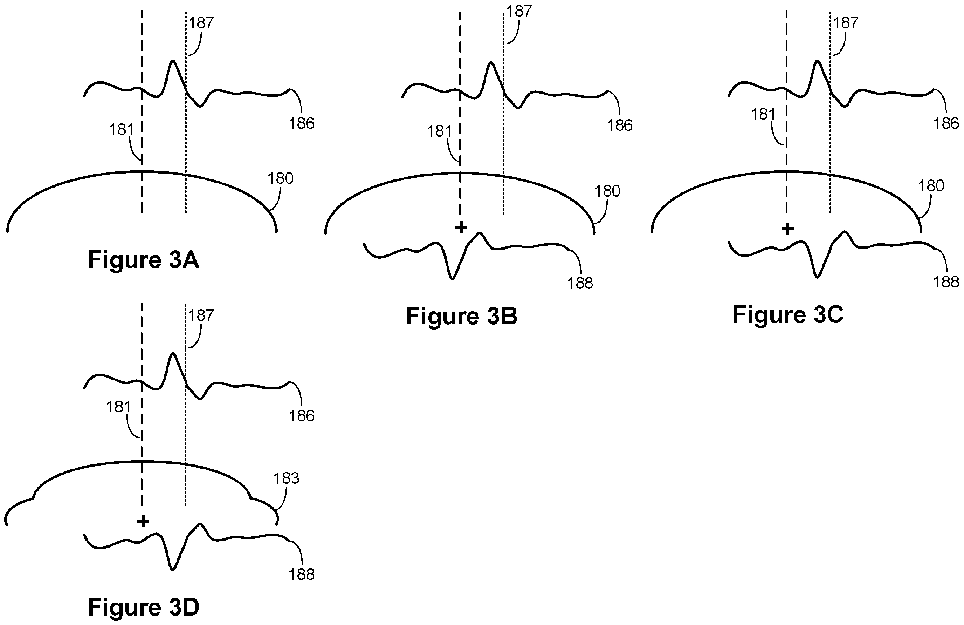

Figures 3A-3D are schematic diagrams illustrating correction of higher-order aberrations in accordance with some embodiments.

Figure 3A illustrates a surface profile of a contact lens 180 without higher-order correction. As a result, an eye wearing the contact lens 180 may see higher-order aberrations represented by line 186. The visual axis 187 of the eye is typically not aligned with the centerline 181 of the contact lens 180, and thus, the measured higher-order aberrations are not aligned with the center of the contact lens 180.

Figure 3B illustrates modification of the surface profile of the contact lens 180 by superposing a surface profile 188 configured to compensate for the higher-order aberrations. However, when the surface profile 188 is positioned around the centerline 181 of the contact lens 180 as shown in Figure 3B, the combined surface profile is not effective in reducing the higher-order aberrations, as the surface profile 188 is offset from the higher-order aberrations measured along the visual axis 187 of the eye.

Figure 3C illustrates modification of the surface profile of the contact lens 180 by superposing the surface profile 188 configured to compensate for the higher-order aberrations where the surface profile 188 is positioned around the visual axis 187 of the eye instead of the centerline 181 of the contact lens 180. By modifying the surface profile of the contact lens 180 by superposing the surface profile 188 with an offset (e.g., the surface profile 188 is in line with the visual axis 187 of the eye), a lens with the modified surface profile can better compensate for higher-order aberrations.

Figure 3D is similar to Figure 3C except that the modification of the surface profile can be applied to a multifocal lens 183.

Although Figures 3A-3D are used to illustrate the importance of the position of the contact lens relative to the visual axis, the orientation and tilt of the contact lens relative to the visual axis are also important.

In some cases, a reference lens with markings is used to assist with determination of the lens position. The reference lens, also called a predicate lens, may serves as an indicator of translation with respect to a visual axis of an eye. In some configurations, the reference lens has a same size as a contact lens (e.g., scleral lens). In some configurations, the reference lens has an optical power (e.g., an optical power to compensate for myopia, hyperopia, or presbyopia, and optionally astigmatism). However, the reference lens may not be configured to compensate for higher-order aberrations. Compared to a contact lens, which is designed to be worn by a patient throughout a day, the reference lens is typically designed to be worn temporarily for diagnostic purposes (e.g., while the patient is at a clinic for one or more measurements by a measurement device, such as measurement device 102, which may be used for prescription of a customized contact lens).

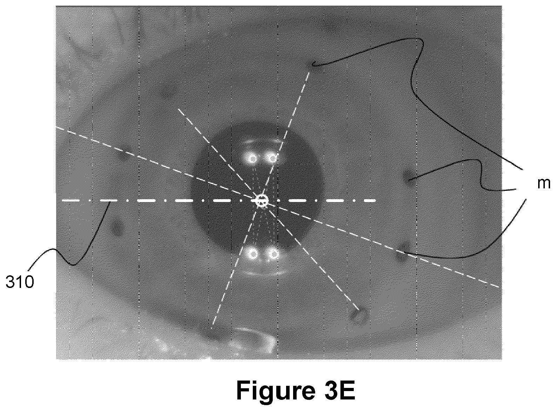

For example, a reference lens with marks, shown in Figure 3E, may be used to determine a position and orientation of the reference lens while the reference lens is positioned on an eye. As shown in Figure 3E, the marks m are arranged in a way so that a center of the lens corresponds to a center of the marks m and an orientation of the lens may be indicated by a rotation of the marks m relative to a reference line 310 (e.g., a horizontal line, a vertical line, or a predefined reference line having a particular orientation).

However, in some cases, when some of the marks are occluded, determination of the orientation and tilt of the lens may be challenging. Thus, there is a need for a lens with marks arranged in a particular pattern that facilitates accurate detection of the orientation and tilt of the lens.

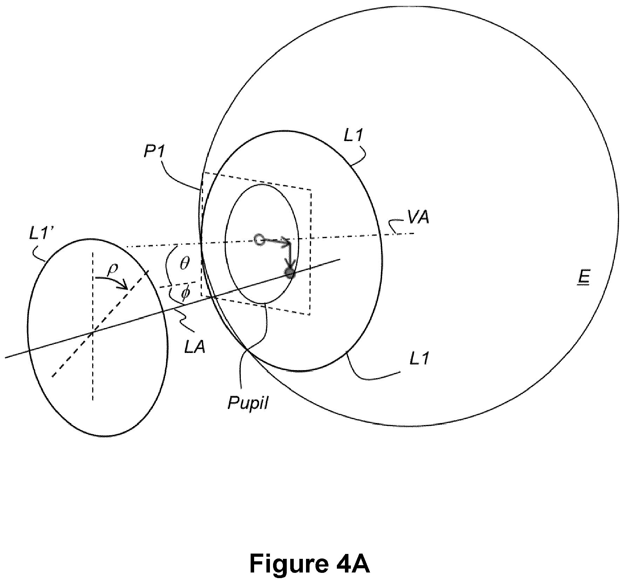

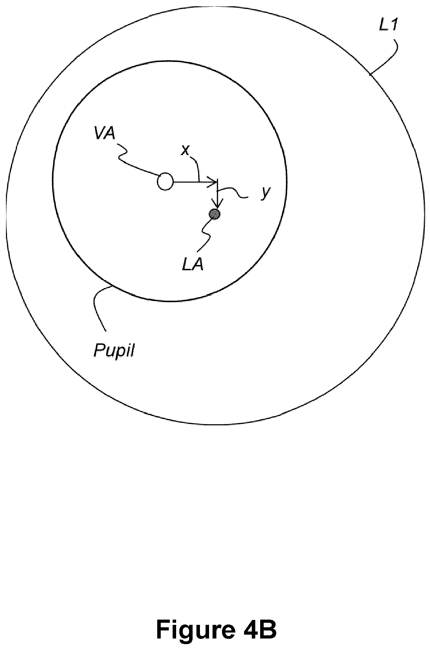

Figure 4A is a schematic diagram illustrating a perspective view of an eye and aspects of lens positioning that relate to design and fitting of the scleral contact lens. Figure 4B is a schematic diagram illustrating a plan view of the eye and the lens shown in Figure 4A, taken along the visual axis (e.g., Figure 4B shows a view of a plane perpendicular to the visual axis).

Coordinates x and y are considered to lie on a plane P1 that is orthogonal to the visual axis VA of the eye E. Angles $\theta$ and $\phi$ relate to orthogonal angular components for skew of the lens axis LA away from visual axis VA.

Although the lens L1 is positioned on a surface of the eye E (e.g., over the cornea and sclera), the lens L1' offset from the surface of the eye E is shown in Figure 4A to illustrate the rotation of the lens L1 without obscuring other aspects of Figure 4A. Angle measurement $\rho$ (also called the orientation) relates to rotation of the lens L1 (e.g., clockwise from a 12 o'clock reference direction). In Figure 4A, the rotation is measured about the lens axis LA. In some cases, the rotation is measured about the visual axis VA of the eye E.

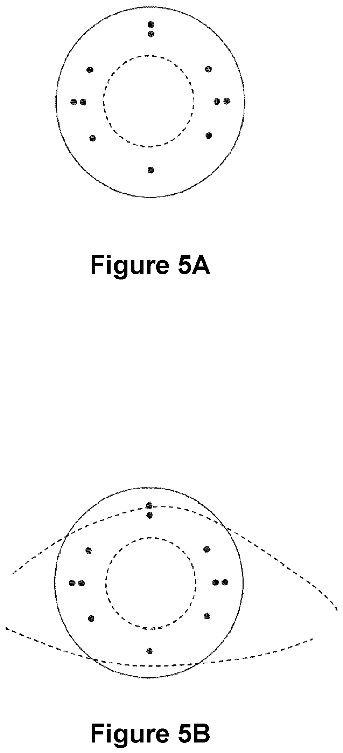

Figure 5A is a schematic diagram illustrating a reference lens with marks in accordance with some embodiments. The reference lens shown in Figure 5A facilitates determining the angular orientation (angle $\rho$ in FIG. 4A). This type of arrangement allows detection of angle even where a portion of the markings are obscured by the patient's eyelid as shown in Figure 5B.

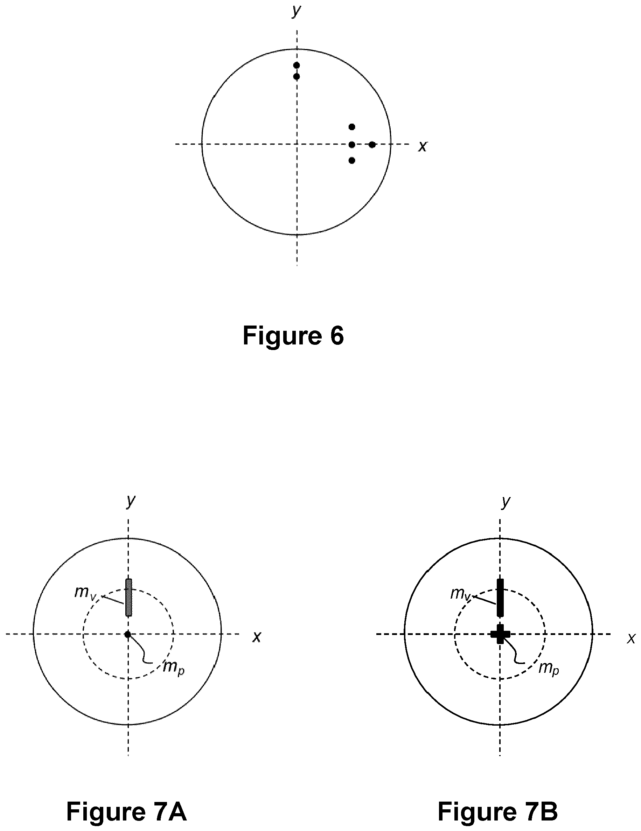

Figure 6 is a schematic diagram illustrating a reference lens with a different marking pattern in accordance with some embodiments. In Figure 6, the lens has two or more marks indicating a position of the lens along a first direction (e.g., along the x-axis) and two or more marks indicating a position of the lens along a second direction (e.g., along the y-axis) that is not parallel to the first direction. Failure to detect one of the marks in either or both clusters is less likely to compromise the ability to determine lens center.

Figure 7A is a schematic diagram illustrating a reference lens with a reduced number of marks in accordance with some embodiments. One mark $m_p$ directly indicates the lens center. The other mark $m_v$ indicates an axis of the lens (e.g., the y-axis). Although Figure 7A shows particular shapes of marks $m_p$ and $m_v$ (a bar and a dot), marks having other shapes may be used. For example, Figure 7B shows a reference lens with mark $m_p$ having a shape of a crosshair and mark $m_v$ having a

shape of a bar.

The position of the lens center (e.g., the x, y translation) and the angular orientation can be determined using any of the arrangements shown in Figures 5A-5B, 6, 7A, and 7B.

In some embodiments, the lenses have marks arranged in a pattern having concentric circles. Such lenses improve accuracy in determination of lens tilt.

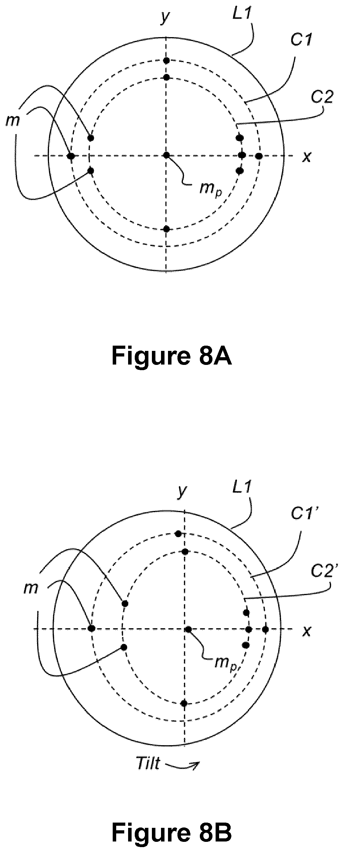

Figures 8A and 8B are front elevational views of an example of a reference lens with marks arranged in a concentric pattern, in accordance with some embodiments.

Figure 8A represents a view of the lens L1 directly along the optical axis (through center point $m_p$) of the lens L1. The circular outer periphery of the scleral contact lens L1 is shown by a continuous line in the Figure 8A. Defined by markings inside the circular outer periphery of lens L1 are an outer circle C1 and an inner circle C2 at longer and shorter radii, respectively, from center point $m_p$. In Figure 8A, each mark m is at one radius or another such that it lies on one circle or another, centered at point $m_p$. In some configurations, marks m can be clustered to show lens rotational orientation, such as in groups of 1, 2, 3, and 4 marks m as shown in Figure 8A. Identifying radii along both x- and y-axes is advantageous for characterizing tilt with respect to both horizontal and vertical references.

Figure 8B represents a view of the lens L1 that is positioned on an eye with a horizontal tilt (about the y-axis), viewed from the same direction used for Figure 8A. Each of circles C1 and C2 can be constructed from the acquired camera image of the predicate lens in place against the eye, with the circles each centered around $m_p$. The use of additional marks helps to identify angular orientation.

Tilt is detectable because the circle is a special case of the more general ellipse. The ellipse has two distinct focus points or foci, one for each of two mutually orthogonal radii. In the circle, focus points for orthogonal radii are the same point, at a single center focus point. Figure 8B shows, in exaggerated form, how the imaged marks m can indicate tilt with respect to the y-axis. Circles C1 and C2 defined by the pattern of marks m shown in Figure 8A are seen as ellipses C1' and C2', respectively, in Figure 8B.

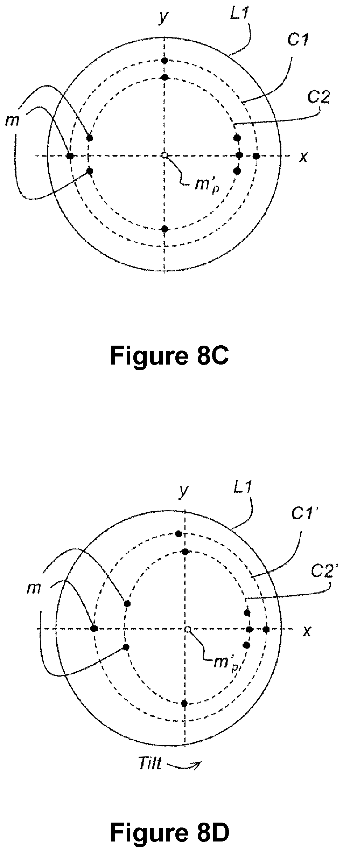

Figures 8C and 8D are schematic diagrams illustrating a reference lens with marks arranged in concentric patterns. The reference lens shown in Figures 8C and 8D are similar to the reference lens shown in Figures 8A and 8B except that the lens does not include a mark $m_p$ for the center of the lens. Instead, the center of the lens may be determined based on the position of other marks m. For example, the lens center can be identified according to geometric construction that defines intersecting orthogonal x- and y-axes, as indicated. Alternatively, other types of geometric construction can be used to find the lens center $m_p$ using markings outside of the center of the lens. Thus, the lens shown in Figures 8C and 8D does not obscure the view of a user, and thus, can improve the accuracy when the position information of the lens is collected in conjunction with the aberrations in the eye. Constructed center point $m'_p$, identified as described in Figures 8C and 8D, can be used in the same manner as the marked point $m_p$.

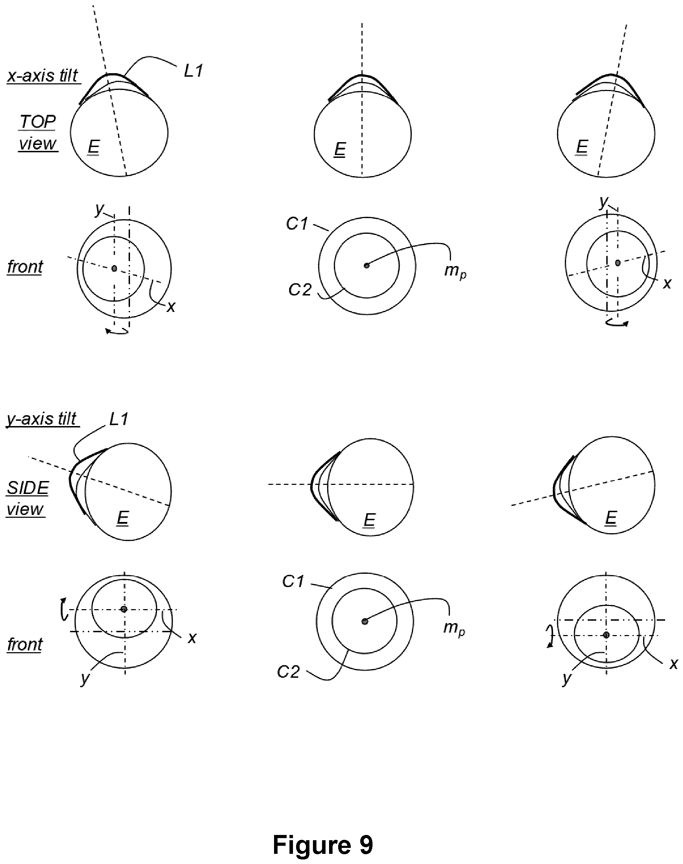

Figure 9 illustrates examples of tilting of the lens. In particular, Figure 9 shows how the arrangement of circles C1 and C2 and their shape transition to ellipses indicate tilt. The top row, with eye E from a top view, indicates horizontal tilt of the lens with respect to eye E about the y-axis, altering the horizontal (x-axis) direction. The second row shows how circles C1 and C2 change in shape and offset to indicate the horizontal tilt. The third row, with eye E from a side view, indicates vertical tilt of the lens with respect to eye E about the x-axis, altering the vertical (y-axis) direction. The fourth row shows how circles C1 and C2 change in shape and offset to indicate the vertical tilt. In essence, circles C1 and C2 become ellipses with tilt of lens L1 even though the eye E is facing the same direction. Tilt is indicated by (i) transition from circular to non-circular elliptical shape, and (ii) variable distance between the ellipses. The changes to radius distance and elliptical shape can be detected by the system and used to closely approximate the tilt of the lens L1 (or the optical axis thereof). Although Figure 9 illustrates horizontal tilt and vertical tilt, in some cases, the lens L1 can have both horizontal tilt and vertical tilt (e.g., tilt about both x- and y-axes).

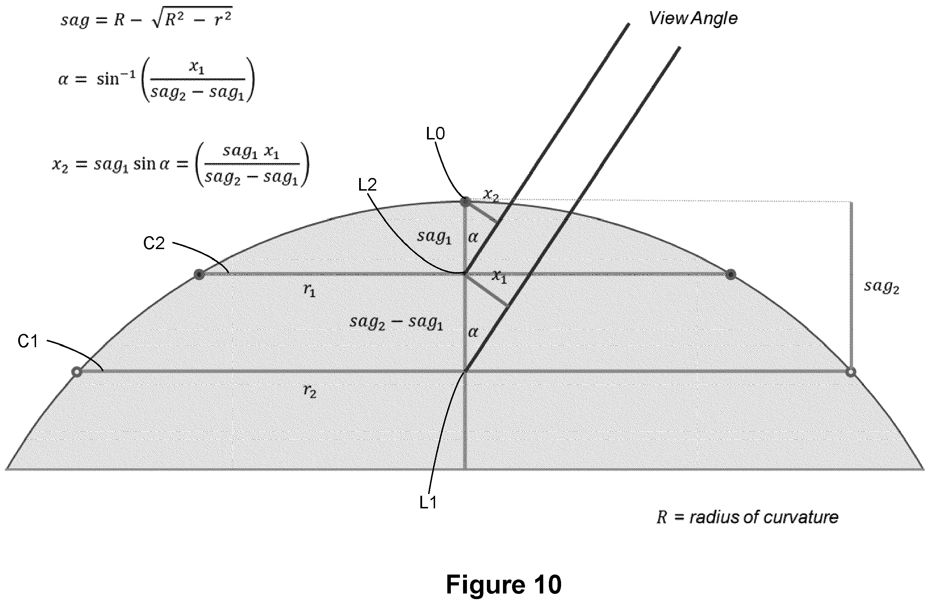

Figure 10 is a schematic diagram illustrating a side view of a lens and a method for determining a tilt of the lens in accordance with some embodiments.

As shown in Figure 9, when the lens is viewed from the tilted direction (e.g., a direction that is non-parallel to an optical axis of the lens), the center L1 of the outer circle C1 and the center L2 of the inner circle C2 are offset from each other. In addition, the center L0 of the lens is also offset from the center L1 of the outer circle C1 and the center L2 of the inner circle C2. Thus, when the lens is tilted relative to the visual axis of the eye (as shown in Figure 8D), the degree of tilt can be determined from the positions of the marks in circles C1 and C2. For example, for a lens having a spherical surface characterized by radius R, a sagitta (also called sag) to a circle (or a plane defined by the circle) having a radius of r is defined as:

$$\mathrm{sag} = \mathrm{R} - (\mathrm{R}^2 - \mathrm{r}^2)^{1/2}$$

Accordingly, $\mathrm{sag}_1$ for the inner circle C2 having the radius $r_1$ and $\mathrm{sag}_1$ for the inner circle C2 having the radius $r_2$ are:

$$\mathrm{sag}_1 = \mathrm{R} - (\mathrm{R}^2 - \mathrm{r}_2{}^2)^{1/2}$$

$$\mathrm{sag}_2 = \mathrm{R} - (\mathrm{R}^2 - \mathrm{r}_1{}^2)^{1/2}$$

Thus, when a reference lens is designed with particular arrangement of the marks on the outer circle C1 (having radius $r_2$) and the inner circle C2 (having radius $r_1$), $\mathrm{sag}_1$ and $\mathrm{sag}_2$ can be determined.

Figure 10 also shows that the perceived offset $x_1$ between the center L1 of the outer circle C1 and the center L2 of the outer circle C2 is:

$$\mathrm{x}_1 = (\mathrm{sag}_2 - \mathrm{sag}_1) \sin \alpha$$

Using this equation, the tilt angle $\alpha$ can be determined from the perceived offset $x_1$ between the center L1 of the outer circle C1 and the center L2 of the outer circle C2.

$$\sin \alpha = \mathrm{x}_1 / (\mathrm{sag}_2 - \mathrm{sag}_1)$$

$$\alpha = \sin^{-1} [\mathrm{x}_1 / (\mathrm{sag}_2 - \mathrm{sag}_1)]$$

Furthermore, the offset $x_2$ between the center L0 of the lens and the center L2 of the inner circle C2 is:

$$\mathrm{x}_2 = \mathrm{sag}_1 \sin \alpha$$

$$\mathrm{x}_2 = \mathrm{x}_1 \mathrm{sag}_1 / (\mathrm{sag}_2 - \mathrm{sag}_1)$$

Thus, by utilizing marks located on two concentric circles, the tilt angle $\alpha$ and the center of the lens L0 can be determined.

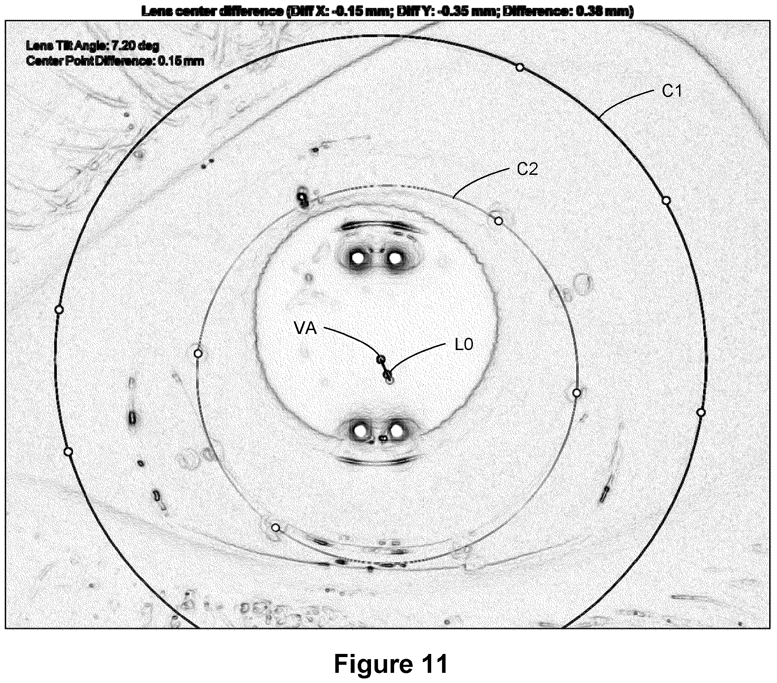

Figure 11 is a front view showing exemplary lens positioning on an eye. Based on the positions of the marks on the outer circle C1 and the positions of the marks on the inner circle C2, the centers of the outer circle C1 and the inner circle C2 were determined. Also the tilt angle $\alpha$ and the position of the center of the lens L0 were determined as described above with respect to Figure 10. In turn, the offset between the visual axis and the center L0 of the lens was determined. A lens profile to compensate for the offset can be designed, as described above with respect to Figures 3A-3C.

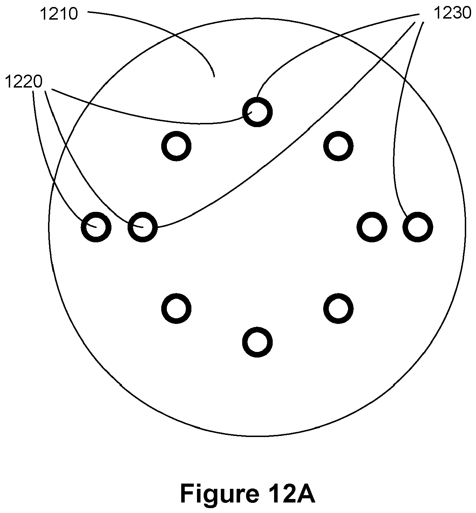

Figure 12A is a schematic diagram illustrating a reference lens 1210 with marks in accordance with some embodiments.

In some embodiments, one or more marks are physical marks. For example, one or more portions 1220 of the reference lens 1210 are removed (e.g., by machining, such as drilling, cutting, milling, etc., etching, or any other patterning method) to define marks. Alternatively, one or more portions of the reference lens 1210 are surface-treated to define marks (e.g., providing a certain surface texture or applying a reflective coating). Physical marks may be placed accurately at predefined positions of the reference lens 1210, and thus, can improve the accuracy in determining the position of the reference lens 1210 while the reference lens 1210 is positioned on an eye.

In some embodiments, one or more marks are highlighted marks (also called color-contrast marks). For example, one or more portions 1230 of the reference lens 1210 are highlighted with a dye (e.g., a visible color dye, a fluorescence dye, an infrared dye, etc.). The highlighted marks may have an optical characteristic (e.g., color) that is distinct from the corresponding optical characteristic of the rest of the lens 1210. For example, the highlighted marks are opaque, which contrasts from the transparent lens 1210. In addition, the highlighted marks may have an optical characteristic (e.g., color) that is distinct from the corresponding optical characteristic of the eye. For example, the highlighted marks have a color that is distinguishable from the color of the eye (e.g., the color of the eye or the color of the sclera, depending on which portions of the eye the marks are expected to be placed over). In some embodiments, the one or more portions 1230 of the reference lens 1210 are highlighted with light scattering material. The highlighted marks facilitate the detection of the marks.

In some embodiments, a combination of the physical marks and highlighted marks are used. For example, as shown in Figure 12A, the reference lens 1210 may have physical marks 1220 (e.g., predefined throughholes or indentations, such as semi-spherical depression) in conjunction with highlighted marks 1230 (e.g., each highlighted mark 1230 surrounding a corresponding physical mark 1220). Such combination improves the accuracy in positioning the marks as well as the visibility of the marks. In some embodiments, the highlighting may be added after the physical marks are formed on the reference lens 1210 (e.g., by adding a dye or an ink to a surface of the reference lens 1210 adjacent to physical marks 1220). In some cases, the physical marks 1220 define wells in which dyes or inks are pooled, allowing accurate positioning of the highlighted marks. In some embodiments, the reference lens 1210 has highlighted marks before the physical marks are formed.

Although marks of different sizes may be used, small marks can improve the accuracy in determining the position and orientation of the lens. The use of the combination of the physical marks and highlighted marks enable use of small marks, while maintaining the ability to detect such marks.

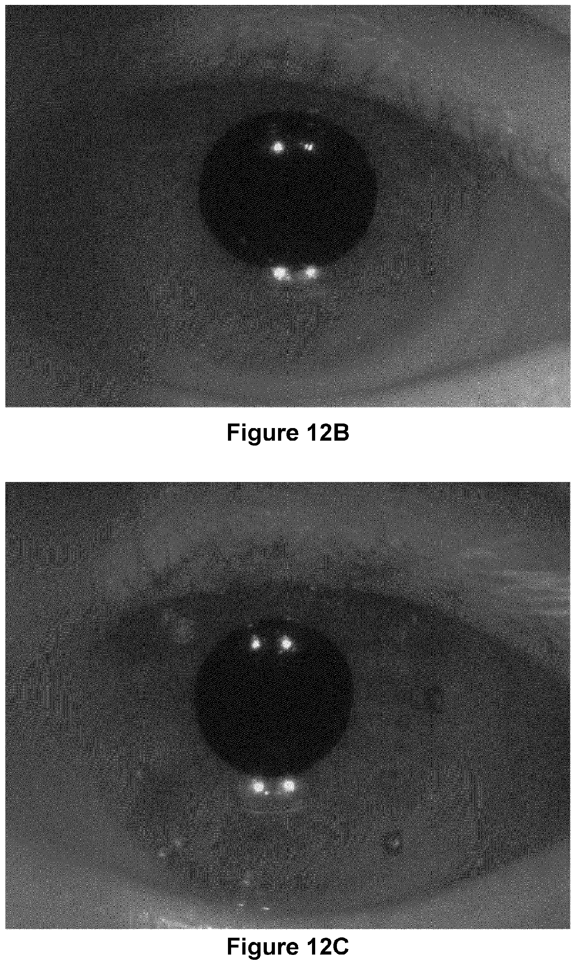

Figure 12B shows a lens with physical marks, positioned on an eye, in accordance with some embodiments. Without the highlighted marks, the physical marks may be difficult to detect within an image of the eye wearing the reference lens with physical marks.

Figure 12C shows a lens with a combination of the physical marks and highlighted marks, positioned on an eye, in accordance with some embodiments. Comparison of Figures 12B and 12C show that the combination of the physical marks and highlighted marks is easier to detect compared to using the physical marks without highlighted marks.

Although Figures 8A-8D and 9-11 illustrate reference lenses with marks arranged around two concentric circles, in some embodiments, the circular periphery of the predicate lens is used for relative measurement of elliptical shape, in combination with one or more circular shapes centered at lens center $m_p$. For example, a reference lens may have distinct and separate marks around one circle, and instead of having additional separate marks around another circle, the periphery of the reference lens may be used to indicate the outer circle. In some embodiments, the periphery of the reference lens is highlighted.

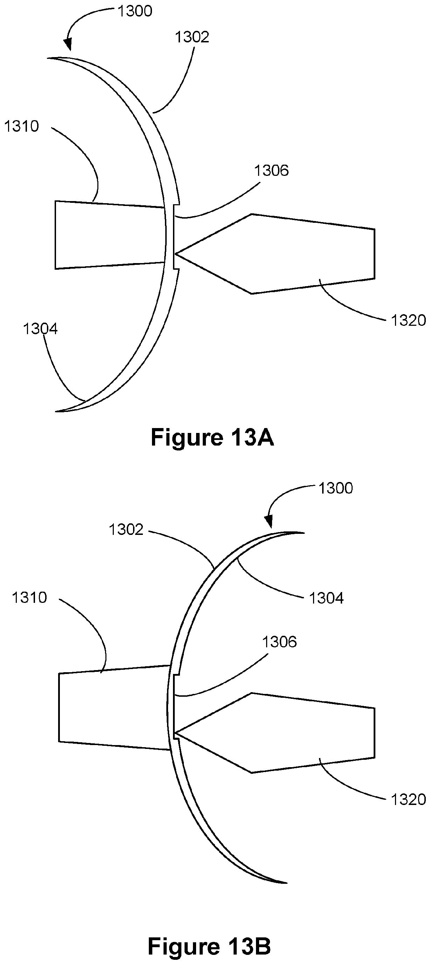

Figures 13A and 13B are schematic diagrams illustrating methods of making a contact lens with one or more indentations in accordance with some embodiments. In some embodiments, instead of, or in addition to, using a highlighted mark, an indentation mark or a scribed mark is used. For example, as shown in Figures 13A and 13B, a portion of contact lens 1300 having anterior surface 1302 (e.g., a convex surface) and posterior surface 1304 (e.g., a concave surface) is marked, removed, or scratched with a machine tool 1320 (e.g., a scriber, a tip of a drill bit, etc.). In some cases, contact lens 1300 is held by chuck 1310 while a portion of contact lens 1300 is removed with machine tool 1320 to form indentation 1306. In some embodiments, indentation 1306 has a smooth surface. In some embodiments, indentation 1306 has a rough surface to increase scattering of light. In some embodiments, anterior surface 1302 of contact lens 1300 is marked, removed, or scratched to form an indentation mark, as shown in Figure 13A. In some embodiments, posterior surface 1304 of contact lens 1300 is marked, removed, or scratched to form an indentation mark, as shown in Figure 13B. In some embodiments, both anterior surface 1302 and posterior surface 1304 of contact lens 1300 are marked, removed, or scratched to form one or more indentation marks.

Eliminating the need for a highlighted mark simplifies the manufacturing process and obviates safety concerns that may be associated with a dye, depending on the type of the dye. In addition, the indentation mark may be used in a reference contact lens (e.g., a predicate lens) as well as in a final contact lens. This allows accurate measurement of the position and rotation of the final contact lens, which can be used for confirmation. Furthermore, an indentation mark without highlighting may be positioned within the field of view of a user (e.g., within a portion of a contact lens that corresponds to a pupil region of an eye of a wearer, such as a diameter having 8 mm, 7 mm, 6 mm, 5 mm, 4 mm, or 3 mm, or within a range between any two of the aforementioned diameters, at a center of the contact lens), because the indentation mark does not significantly interfere with the view of the wearer due to the absence of a dye.

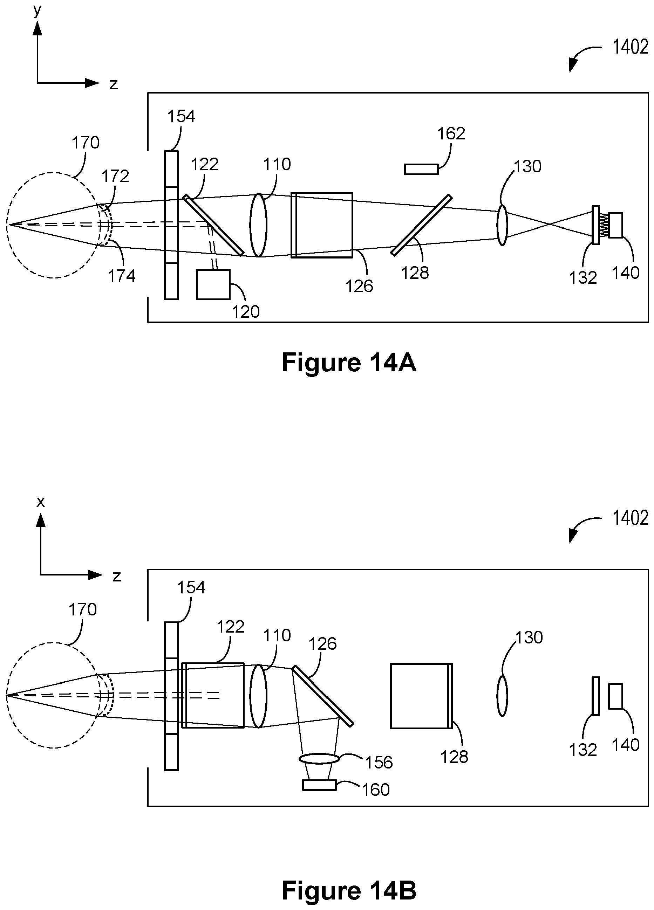

Figures 14A and 14B illustrate wavefront sensing and mark imaging with optical device 1402 in accordance with some embodiments. Optical device 1402 is similar to optical device 102 described with respect to Figures 1B-1E and optical device 103 described with respect to Figures IF and 1G. Thus, optical device 1402 may perform wavefront sensing as shown in Figure 14A. However, beam steerer 126 of optical device 1402 is configured to reflect a portion of light from eye 170 toward image sensor 160 and transmit a portion of light from eye 170 toward image sensor 140 (e.g., beam steerer 126 is a partial reflector, such as a 50:50 mirror).

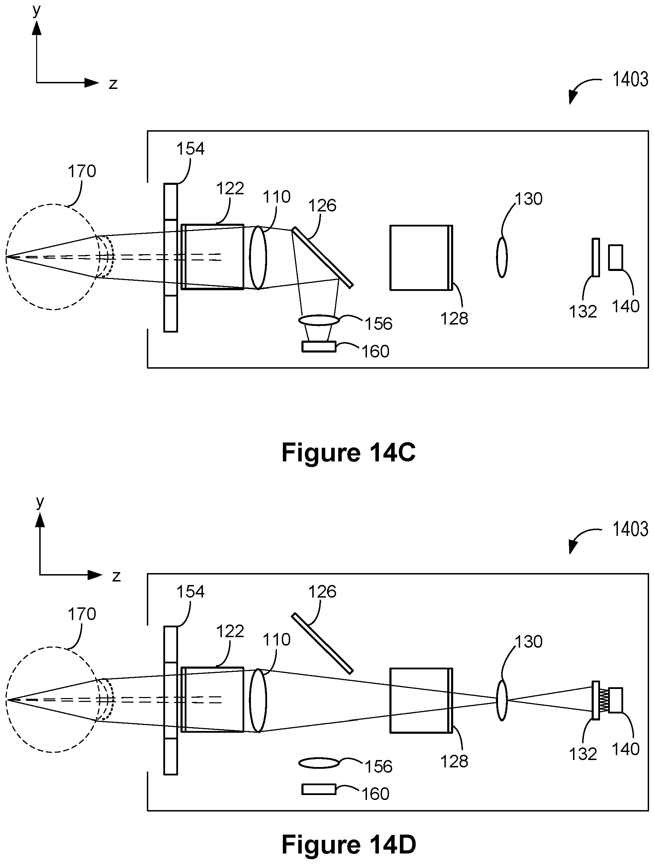

Figures 14C and 14D illustrate optical device 1403, which is similar to optical device 1402 described with respect to Figures 14A and 14B. However, beam steerer 126 of optical device 1403 is a movable reflector (e.g., a reflector configured to move, such as rotate and/or translate). As shown in Figure 14C, while beam steerer 126 is positioned within a path of light from lens 110, beam steerer 126 directs the light toward image sensor 160 (e.g., through lens 156). Figure 14D illustrates that while beam steerer 126 is positioned out of the path of light from lens 110, the light propagates toward the array of lenses 132 and image sensor 140.

These configurations shown in Figures 14A-14D allow image sensor 160 to receive light from eye 170 illuminated with light from first light source 120. In some cases, as shown in Figures 14A-14D, light from light source 120 impinges on a retina of eye 170 and is returned back toward the optical device (e.g., optical device 1402 or 1403) via back reflection or back scattering. The back reflection or back scattering facilitate identifying indentation marks on a contact lens positioned on eye 170. As a result, an image obtained based on the light returned from the eye allows determining positions of marks on a contact lens 174 even when the marks do not include highlighting (e.g., indentation marks not highlighted with a dye).

In some embodiments, optical device 1402 does not include second light source 154. In some embodiments, optical device 1402 is also capable of imaging eye 170 illuminated with light from second light source 154 (e.g., using image sensor 160) in addition to obtaining an image from the light received from eye 170 in response to illumination with light from first light source 120. For example, optical device 1402 may be configured to illuminate eye 170 with light from first light source 120 at a first time and illuminate eye 170 with light from second light source 154 at a second time that is distinct from the first time, while image sensor 160 may receive light from the eye illuminated with light from first light source 120, light from second light source 154, or both.

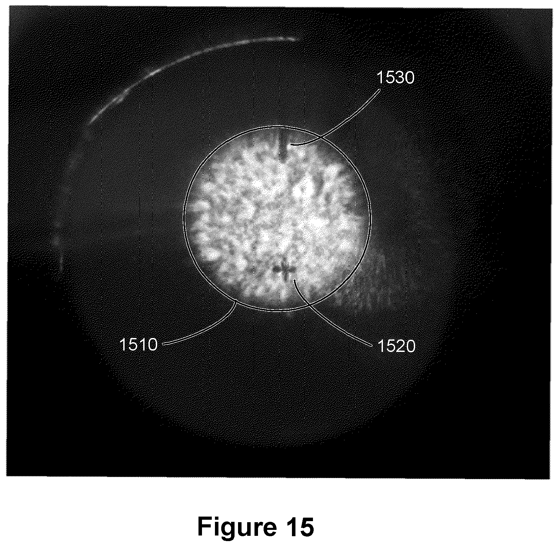

Figure 15 is an image of a contact lens with indentation marks in accordance with some embodiments. The image was obtained based on light received from an eye illuminated with a narrow beam (e.g., a laser beam). The image shown in Figure 15 includes a ring 1510, which corresponds to a pupil of the eye, and shadows 1520 and 1530 of marks, which correspond to $m_p$ and $m_v$ shown in Figure 7B. Although Figure 15 shows an image of a contact lens with the marks corresponding to those shown in Figure 7B, a contact lens with any pattern of marks described herein or any variant thereof may be used.

In light of these principles and examples, we turn to certain embodiments.

Some embodiments include a contact lens with a plurality of marks for indicating a position of the contact lens and a rotation of the contact lens while the contact lens is positioned on an eye of a user (e.g., Figure 7B). In some embodiments, the plurality of marks includes at least one mark that is scribed on the contact lens.

In some embodiments, a contact lens with a plurality of marks for indicating a position of the contact lens and a rotation of the contact lens while the contact lens is positioned on an eye of a user includes at least one indentation mark (e.g., a mark that is formed by scribing on the contact lens, or otherwise removing a portion of the contact lens). In some embodiments, the contact lens with at least one indentation mark is formed by machining a contact lens substrate that includes a preformed indentation (e.g., an indentation for a center of the contact lens).

In some embodiments, the plurality of marks includes a first set of one or more marks for indicating the position of the contact lens (e.g., mark $m_p$ shown in Figure 7B).

In some embodiments, the first set of one or more marks includes a mark positioned adjacent to a center of the contact lens (e.g., mark $m_p$ shown in Figure 7B is positioned at a center of the contact lens).

In some embodiments, the plurality of marks includes a second set of one or more marks for indicating the rotation of the contact lens (e.g., mark $m_v$ shown in Figure 7B).

In some embodiments, the second set of one or more marks includes a mark positioned at least partially within an area corresponding to a pupil of the eye (e.g., mark $m_v$ shown in Figure 7B is positioned within an area of the contact lens corresponding to the pupil of the eye, as shown in Figure 15). For example, at least one mark of the second set of one or more marks is located within 1 mm, 1.5 mm, 2 mm, 2.5 mm, 3 mm, 3.5 mm, 4 mm, 4.5 mm, 5 mm, 5.5 mm, 6 mm, 6.5 mm, 7 mm, 7.5 mm, 8 mm, or within a range between any two of the aforementioned distances, from a center of the contact lens.

In some embodiments, the scribed mark is located on an anterior surface of the contact lens (e.g., Figure 13A). In some embodiments, the plurality of marks includes at least one mark that is scribed on the anterior surface of the lens.

In some embodiments, the scribed mark is located on a posterior surface of the contact lens (e.g., Figure 13B). In some embodiments, the plurality of marks includes at least one mark that is scribed on the posterior surface of the lens.

In some embodiments, the anterior surface of the contact lens includes a region having a convex surface (e.g., Figure 13A).

In some embodiments, the plurality of marks includes at least one mark that is positioned on the contact lens without using a dye.

In accordance with some embodiments, a method of making a contact lens includes obtaining a first contact lens (e.g., a contact lens without one or more marks or a contact lens, to which one or more marks are to be added). The method also includes forming one or more indentations on the first contact lens for indicating a position of the contact lens and a rotation of the contact lens (e.g., Figure 13 A).

In some embodiments, forming the one or more indentations on the first contact lens includes scribing the one or more indentations on the first contact lens.

In some embodiments, obtaining the first contact lens includes obtaining a substrate, and removing one or more portions of the substrate to obtain the first contact lens. For example, a contact lens substrate (e.g., a substrate that includes polymer-hydrogel or silicone-hydrogel) is machined (e.g., lathed) to form a contact lens, before one or more indentations are formed on the contact lens.

In some embodiments, the one or more indentations are formed on an anterior surface of the first contact lens. In some embodiments, the one or more indentations are formed without using a dye.

In accordance with some embodiments, a device (e.g., optical device 1402) includes a light source (e.g., light source 120 shown in Figure 14A) for providing light to an eye so that at least a portion of the light is returned to the device. The device also includes a first image sensor (e.g., image sensor 160 shown in Figure 14A) positioned to image a pupil region of the eye for capturing a shadow of one or more marks on a contact lens positioned on the eye.

In some embodiments, the device also includes a wavefront sensor that includes one or more lenses (e.g., lens 130 shown in Figure 14A) positioned to receive the returned light, an array of lenses (e.g., the array of lenses 132 shown in Figure 14A) that is distinct and separate from the one or more lenses, the array of lenses being positioned to focus at least a portion of the light received by the one or more lenses, and a second image sensor (e.g., image sensor 140 shown in Figure 14A) positioned to receive the light focused by the array of lenses.

In some embodiments, the device also includes a beam steerer (e.g., beam steerer 126 shown in Figures 14A and 14B) positioned to allow at least a portion of the returned light to propagate toward the first image sensor and allow at least a portion of the returned light to propagate toward the second image sensor.

In some embodiments, the beam steerer includes a partial reflector. In some embodiments, the beam steerer is a partial reflector.

In some embodiments, the beam steerer includes a movable reflector. In some embodiments, the beam steerer is a movable reflector.

In some embodiments, the device also includes one or more communication interfaces for sending information based on images obtained by the first image sensor and the second image sensor to an electronic device that is located remotely from the device for fabrication of a contact lens. For example, the device may include one or more components shown in Figure 2, especially communication interface 204, for sending images obtained by the first image sensor and the second image sensor, or information extracted from such images, such as a position and rotation of the contact lens on an eye, as well as wavefront information. Such information is conveyed to a remote electronic device (e.g., a server computer) for operating manufacturing tools (e.g., machining tools) for fabricating customized contact lenses.

In accordance with some embodiments, a method includes providing light to an eye wearing a contact lens with one or more marks (e.g., as shown in Figure 14A, light from light source 120 is provided to an eye wearing a contact lens with one or more marks).

The method also includes receiving at least a portion of the light returned from the eye (e.g., as shown in Figure 14A, back reflection or back scattering is received by lens 110).

The method further includes imaging the received light to form an image with respective shadows of the one or more marks (e.g., as shown in Figure 14A, the received light is directed to image sensor 160 for imaging). For example, as shown in Figure 15, the received light forms an image with shadows 1520 and 1530 of marks $m_p$ and $m_v$.

In some embodiments, the method further includes determining a position and a rotation of the contact lens on the eye based on the image with respective shadows of the one or more marks. Similar to the methods described with respect to Figures 8A-8D, 9, 10, and 11 for determining the position and the rotation of the contact lens on the eye based on the locations of marks in the image, the position and the rotation of the contact lens on the eye can be determined from the locations of the shadows of marks in the image.

Some embodiments are described in terms of the following clauses.

Clause 1. A contact lens, comprising:
an optically transparent lens with a plurality of marks for indicating a position of the contact lens and a rotation of the contact lens while the contact lens is positioned on an eye of a user.
Clause 2. The contact lens of clause 1, wherein the plurality of marks includes a first set of one or more marks for indicating the position of the contact lens.
Clause 3. The contact lens of clause 2, wherein the plurality of marks includes a second set of one or more marks for indicating the rotation of the contact lens.

Clause 4. The contact lens of clause 2 or 3, wherein the first set of one or more marks includes a mark positioned at a center of the contact lens.

Clause 5. The contact lens of any of clauses 2-4, wherein the first set of one or more marks includes one or more marks positioned at a first distance from a center of the contact lens.

Clause 6. The contact lens of clause 5, wherein the second set of one or more marks includes one or more marks positioned at a second distance, distinct from the first distance, from the center of the contact lens.

Clause 7. The contact lens of any of clauses 3-6, wherein the first set of one or more marks and the second set of one or more marks are mutually exclusive.

Clause 8. The contact lens of any of clauses 3-6, wherein the first set of one or more marks and the second set of one or more marks have at least one common mark.

Clause 9. The contact lens of clause 8, wherein the first set of one or more marks includes at least one mark that is not included in the second set of one or more marks or the second set of one or more marks includes at least one mark that is not included in the first set of one or more marks.

Clause 10. The contact lens of any of clauses 2-9, wherein the plurality of marks also includes a third set of one or more marks for indicating a tilt of the contact lens while the contact lens is positioned on the eye of the user.

Clause 11. The contact lens of clause 10, wherein the third set of one or more marks includes one or more marks positioned at a third distance, distinct from the first distance, from a center of the contact lens.

Clause 12. The contact lens of clause 11, wherein the third distance is different from the second distance.

Clause 13. The contact lens of clause 11, wherein the third distance is identical to the second distance.

Clause 14. The contact lens of any of clauses 1-13, wherein the contact lens includes one or more quadrants without any of the plurality of marks.

Clause 15. The contact lens of any of clauses 1-14, wherein the contact lens includes one or more quadrants without any of the plurality of marks.

Clause 16. The contact lens of any of clauses 1-15, wherein each mark of the plurality of marks has a circularly symmetric shape.

Clause 17. The contact lens of any of clauses 1-15, wherein at least one of the plurality of marks has a shape that is not circularly symmetric.

Clause 18. A contact lens, comprising:
an optically transparent lens with a plurality of marks, the plurality of marks including:

a first set of one or more marks including one or more marks positioned at a first distance from a center of the contact lens; and
a second set of one or more marks including one or more marks positioned at a second distance, distinct from the first distance, from the center of the contact lens.

Clause 19. The contact lens of any of clauses 1-18, wherein the lens has a lens center identifiable according to one or more marks of the plurality of marks.

Clause 20. The contact lens of any of clauses 1-19, wherein one or more marks of the plurality of marks include a fluorescent material.

Clause 21. A method, comprising:

obtaining, with an optical device, information indicating at least one of: a position and
a rotation of the contact lens of any of clauses 1-20 on an eye wearing the contact lens.

Clause 22. The method of clause 21, including obtaining information indicating both of the position and the rotation of the contact lens on the eye wearing the contact lens.

Clause 23. The method of clause 21 or 22, further comprising:
determining at least one of: the position and the rotation of the contact lens on the eye wearing the contact lens.

Clause 24. The method of any of clauses 21-23, further comprising:
obtaining information indicating a tilt of the contact lens on the eye wearing the contact lens.

Clause 25. The method of any of clauses 21-24, further comprising:
determining a tilt of the contact lens on the eye wearing the contact lens.

Clause 26. The method of any of clauses 21-25, further comprising:
obtaining, with the optical device, information indicating one or more aberrations of the eye wearing the contact lens.

Clause 27. The method of clause 26, including:
obtaining, with the optical device, information indicating one or more aberrations of a combination of the eye and the contact lens.

Clause 28. The method of clause 26 or 27, wherein the information indicating the one or more aberrations of the eye includes information indicating one or more high-order aberrations of the eye.

Clause 29. The method of any of clauses 26-28, wherein:
obtaining the information indicating the one or more aberrations of the eye includes conducting one or more of: wavefront sensing, corneal topography, optical coherence tomography, interferometry, wavefront curvature sensing, Foucault knife-edge testing, Ronchi testing, and phase-shifting Schlieren testing.

Clause 30. The method of any of clauses 26-29, including:

> presenting a target image to the eye; and
> while the target image is presented to the eye:
>
>> obtaining, with the optical device, the information indicating the one or more aberrations of the eye; and
>> obtaining, with the optical device, the information indicating one or more of the visual axis of the eye, the center of the pupil of the eye, and the corneal vertex.

Clause 31. The method of any of clauses 21-30, further comprising:
obtaining, with the optical device, information indicating one of more of: a visual axis of the eye, a center of a pupil of the eye, and a corneal vertex of the eye.

Clause 32. The method of clause 31, wherein the information indicating the one or more aberrations of the eye and the information indicating the one or more of the visual axis of the eye, the center of the pupil of the eye, and the corneal vertex of the eye are obtained concurrently.

Clause 33. The method of clause 31 or 32, wherein: obtaining the information indicating the one or more of the visual axis of the eye, the center of the pupil of the eye, and the corneal vertex of the eye includes illuminating the eye with a predefined pattern of light and collecting an image of the eye while the eye is illuminated with the predefined pattern of light.

Clause 34. The method of any of clauses 31-33, further comprising at least one of:

> determining the visual axis of the eye from the information indicating the visual axis of the eye;
> determining the center of the pupil of the eye from the information indicating the center of the pupil of the eye; and
> determining the corneal vertex of the eye from the information indicating the corneal vertex of the eye.

Clause 35. The method of any of clauses 31-34, further comprising:
storing the information indicating the one or more aberrations of the eye in conjunction with the information indicating the one or more of the visual axis of the eye, the center of the pupil of the eye, and the corneal vertex of the eye.

Clause 36. The method of any of clauses 31-35, further comprising:
transferring the information indicating the one or more aberrations of the eye in conjunction with the information indicating the one or more of the visual axis of the eye, the center of the pupil of the eye, and the corneal vertex of the eye.

Clause 37. An optical device, comprising:

> an aberrometer;
> a first light source for providing first light toward an eye;
> a lens assembly for collecting light from the eye; and
> a first image sensor for receiving light that has been transmitted through the lens assembly.

Clause 38. The optical device of clause 37, wherein: the aberrometer is configured to determine aberrations of the eye, including high-order aberrations of the eye.

Clause 39. The optical device of clause 37 or 38, wherein:

> the light source includes a plate with a window and a plurality of light emitting elements arranged on the plate;
> the aberrometer is positioned to measure one or more aberrations of the eye through the window of the plate; and
> the lens assembly and the first image sensor are positioned to collect an image of the eye through the window of the plate.

Clause 40. The optical device of any of clauses 37-39, wherein:
the aberrometer comprises one or more of: a wavefront sensor, a corneal topographer, an optical coherence tomographer, an interferometer, a wavefront curvature sensor, a Foucault knife-edge tester, a Ronchi tester, and a phase-shifting Schlieren tester.

Clause 41. The optical device of any of clauses 37-40, wherein:
the aberrometer is a wavefront sensor that includes:

> a second light source configured to emit second light and transfer the second light toward the eye;
> the lens assembly;
> an array of lenses that is distinct and separate from the lens assembly, the array of lenses configured to focus light transmitted through the lens assembly; and
> a second image sensor for receiving light transmitted through the lens assembly and the array of lenses.

Clause 42. The optical device of any of clauses 37-41, further comprising:

> one or more processors; and

memory storing one or more programs for execution by the one or more processors, the one or more programs including instructions for:

determining one or more aberrations of the eye based on information corresponding to light received by the aberrometer; and determining one or more of a visual axis of the eye, a center of a pupil of the eye, and a corneal vertex of the eye based on information corresponding to the light received by the first image sensor.

Clause 43. The optical device of clause 42, wherein: the one or more programs include instructions for: determining a position of a contact lens relative to the eye from information corresponding to the light received by the second image sensor while the contact lens is positioned adjacent to the eye.

Clause 44. The optical device of clause 42 or 43, wherein: the one or more programs include instructions for: determining a rotation of a contact lens relative to the eye from information corresponding to the light received by the second image sensor while the contact lens is positioned adjacent to the eye.

Clause 45. The optical device of any of clauses 42-44, wherein: the one or more programs include instructions for:

storing the information corresponding to the light received by the aberrometer; and storing the information corresponding to the light received by the first image sensor.

Clause 46. The optical device of any of clauses 42-45, wherein: the one or more programs include instructions for:

transferring the information corresponding to the light received by the aberrometer; and transferring the information corresponding to the light received by the first image sensor.

Clause 47. The optical device of any of clauses 37-46, further comprising: a target for presenting an image of the target to the eye.

Clause 48. A method for making a personalized contact lens, the method comprising:

selecting a surface profile based on (i) one or more aberrations of an eye and (ii) a position of a contact lens relative to one or more of a visual axis of the eye, a center of a pupil of the eye, and a corneal vertex of the eye; and fabricating a contact lens having the selected surface profile.

Clause 49. The method of clause 48, wherein: the surface profile is selected also based on a rotation of the contact lens on the eye.

Clause 50. The method of clause 48 or 49, including:

obtaining a first surface profile corresponding to one or more low-order aberrations of the eye; obtaining a second surface profile for compensating for one or more high-order aberrations of the eye; offsetting the second surface profile based on the position of the contact lens relative to the one or more of the visual axis of the eye, the center of the pupil of the eye, and the corneal vertex of the eye; and superposing the first surface profile and the offset second surface profile to obtain the selected surface profile.

Clause 51. The method of any of clauses 48-50, wherein: fabricating the contact lens having the selected surface profile includes one or more of: cutting or molding.

Clause 52. A contact lens made by the method of any of clauses 48-51.

Clause 53. The contact lens of clause 52, including:

a first region corresponding a first optical power; and a second region corresponding a second optical power that is different from the first optical power.

Clause 54. The contact lens of clause 53, wherein the second region encircles the first region.

Clause 55. A method for making a personalized multifocal lens, the method comprising:

selecting a surface profile based on (i) one or more aberrations of an eye and (ii) a position of a contact lens relative to one or more of a visual axis of the eye, a center of a pupil of the eye, and a corneal vertex of the eye of the eye, wherein an optical zone of the surface profile is positioned based on the position of the contact lens relative to the one or more of the visual axis of the eye, the center of the pupil of the eye, and the corneal vertex of the eye, wherein the surface profile includes a first region corresponding to a first optical power and a second region corresponding a second optical power that is different from the first optical power; and fabricating a contact lens having the selected surface profile.

Clause 56. The method of clause 55, wherein: the surface profile is selected also based on a rotation of the contact lens on the eye.

Clause 57. A multifocal contact lens made by the method of clause 55 or 56.

Clause 58. A method, comprising:

determining one or more aberrations of a combination of an eye and the contact lens of any of clauses 1-20 positioned adjacent to the eye and determining a location of a center of the contact lens by using a single optical device that includes:

a lens assembly;
a wavefront sensor that includes:

a first light source configured to emit first light and transfer the first light emitted from the first light source toward the combination of the eye and the contact lens;
the lens assembly for collecting light from the combination of the eye and the contact lens;
an array of lenses that is distinct from the lens assembly, the array of lenses configured to focus light transmitted through the lens assembly; and
a first image sensor configured to receive light, from the combination of the eye and the contact lens, transmitted through the lens assembly and the array of lenses; and

a contact lens center sensor that includes:

a second light source configured to emit second light and transfer the second light emitted from the second light source toward the combination of the eye and the contact lens;
the lens assembly for collecting light from the combination of the eye and the contact lens;
one or more lenses configured to focus light transmitted through the lens assembly, the one or more lenses being distinct and separate from the array of lenses; and
a second image sensor configured to receive light, from the combination of the eye and the contact lens, transmitted through the lens assembly and the one or more lenses.

Clause 59. The method of clause 58, including:

determining the one or more aberrations of the combination of the eye and the contact lens based on information corresponding to the light received by the first image sensor; and
determining the location of the center of the contact lens based on information corresponding to the light received by the second image sensor.

Clause 60. The method of clause 58 or 59, including:
concurrently transferring the first light emitted from the first light source toward the combination of the eye and the contact lens while transferring the second light emitted from the second light source toward the combination of the eye and the contact lens.

Clause 61. The method of clause 60, including:
projecting an image of a reference toward the eye concurrently with transferring the first light emitted from the first light source toward the combination of the eye and the contact lens and transferring the second light emitted from the second light source toward the combination of the eye and the contact lens.

Clause 62. The method of any of clauses 58-61, including:
concurrently receiving light with the first image sensor while receiving light with the second image sensor.

Clause 63. The method of clause 58 or 59, including:
sequentially transferring the first light emitted from the first light source toward the combination of the eye and the contact lens and transferring the second light emitted from the second light source toward the combination of the eye and the contact lens.

Clause 64. The method of any of clauses 58-61 and 63, including:
sequentially receiving light with the first image sensor and receiving light with the second image sensor.

Clause 65. The method of any of clauses 58-64, wherein:

the first light has a first pattern when projected on the combination of the eye and the contact lens; and
the second light has a second pattern when projected on the combination of the eye and the contact lens.

Clause 66. The method of clause 65, wherein:

the first pattern includes a first array of spots; and/or
the second pattern includes a second array of spots.

Clause 67. The method of any of clauses 58-66, wherein:
the location of the center of the contact lens is determined based on information representing to the light received by the second image sensor.

Clause 68. The method of any of clauses 58-67, further comprising:

storing information representing the light received by the first image sensor; and
storing information representing the light received by the second image sensor in conjunction with the information representing the light received by the first image sensor.

Clause 69. The method of any of clauses 58-68, further comprising:

storing information representing the light received by the first image sensor; and
storing information representing the location of the center of the contact lens in conjunction with the information representing the light received by the first image sensor.

Clause 70. A method, comprising:

transferring first light from a first light source toward a combination of an eye and the contact lens of any of clauses 1-20 positioned adjacent to the eye;
receiving, with a first image sensor, light from the combination of the eye and the contact lens, transmitted through a lens assembly and an array of lenses;
determining one or more aberrations of the combination of the eye and the contact lens;
transferring second light from a second light source toward the combination of the eye and the contact lens;
receiving, with a second image sensor distinct and separate from the first image sensor, light from the combination of the eye and the contact lens, transmitted through the lens assembly and one or more lenses; and
determining a location of a center of the contact lens.

Clause 71. An optical device, comprising:

a lens assembly;
a wavefront sensor that includes:

a first light source configured to emit first light and transfer the first light emitted from the first light source toward a combination of an eye and the contact lens of any of clauses 1-20 positioned adjacent to the eye;
the lens assembly for collecting light from the combination of the eye and the contact lens;
an array of lenses that is distinct from the lens assembly, the array of lenses configured to focus light transmitted through the lens assembly; and
a first image sensor configured to receive light, from the combination of the eye and the contact lens, transmitted through the lens assembly and the array of lenses;

a contact lens center sensor that includes:

a second light source configured to emit second light and transfer the second light emitted from the second light source toward the combination of the eye and the contact lens;
the lens assembly for collecting light from the combination of the eye and the contact lens;
one or more lenses configured to focus light transmitted through the lens assembly, the one or more lenses being distinct and separate from the array of lenses; and
a second image sensor configured to receive light, from the combination of the eye and the contact lens, transmitted through the lens assembly and the one or more lenses;

one or more processors; and
memory storing one or more programs for execution by the one or more processors, the one or more programs including instructions for:

determining one or more aberrations of the combination of the eye and the contact lens based on information corresponding to the light received by the first image sensor; and
determining a location of a center of the contact lens based on information corresponding to the light received by the second image sensor.

Clause 72. The device of clause 71, wherein:
the one or more programs include instructions for:

storing information representing the light received by the first image sensor; and
storing information representing the light received by the second image sensor.

Clause 73. The device of clause 71 or 72, including:
a beam steerer configured to transfer light from the combination of the eye and the contact lens, transmitted through the lens assembly, toward the first image sensor and/or the second image sensor.
Clause 74. The device of clause 73, wherein the beam steerer is a beam splitter.
Clause 75. A method for making a contact lens, the method comprising:

selecting a surface profile based on (i) one or more aberrations of a combination of an eye and a contact lens positioned adjacent to the eye and (ii) a location of a center of the contact lens; and
making a lens having the selected surface profile.

Clause 76. The method of clause 75, including: adjusting the one or more aberrations of the combination of the eye and the contact lens based on the location of a visual axis of the eye.

Clause 77. The method of clause 75, including:

modifying information representing light received by a first image sensor based on information representing light received by a second image sensor; and
determining the one or more aberrations of the eye based on the modified information.

Clause 78. A contact lens made by any method of clauses 75-77.

Clause 79. A method for making a personalized multifocal lens, the method comprising:

selecting a surface profile based on one or more aberrations of an eye, wherein the one or more aberrations of the eye includes one or more high-order aberrations of the eye; and
fabricating a contact lens having the selected surface profile.

Clause 80. A contact lens with a plurality of marks for indicating a position of the contact lens and a rotation of the contact lens while the contact lens is positioned on an eye of a user, wherein the plurality of marks includes at least one mark that is scribed on the contact lens.

Clause 81. The contact lens of claim 80, wherein the plurality of marks includes a first set of one or more marks for indicating the position of the contact lens.

Clause 82. The contact lens of claim 81, wherein the first set of one or more marks includes a mark positioned adjacent to a center of the contact lens.

Clause 83. The contact lens of any of claims 80-82, wherein the plurality of marks includes a second set of one or more marks for indicating the rotation of the contact lens.

Clause 84. The contact lens of claim 83, wherein the second set of one or more marks includes a mark positioned at least partially within an area corresponding to a pupil of the eye.

Clause 85. The contact lens of any of claims 80-84, wherein the scribed mark is located on an anterior surface of the contact lens.

Clause 86. The contact lens of claim 85, wherein the anterior surface of the contact lens includes a region having a convex surface.

Clause 87. The contact lens of any of claims 80-86, wherein the plurality of marks includes at least one mark that is positioned on the contact lens without using a dye.

Clause 88. A method of making the contact lens of any of claims 80-87, the method comprising:

obtaining a first contact lens; and
forming one or more indentations on the first contact lens for indicating a position of the contact lens and a rotation of the contact lens.

Clause 89. The method of claim 88, wherein forming the one or more indentations on the first contact lens includes scribing the one or more indentations on the first contact lens.

Clause 90. The method of claim 88 or 89, wherein obtaining the first contact lens includes:

obtaining a substrate; and
removing one or more portions of the substrate to obtain the first contact lens.

Clause 91. The method of any of claims 88-90, wherein:
the one or more indentations are formed on an anterior surface of the first contact lens.

Clause 92. A device, comprising:

a light source for providing light to an eye so that at least a portion of the light is returned to the device; and
a first image sensor positioned to image a pupil region of the eye for capturing a shadow of one or more marks on a contact lens positioned on the eye.

Clause 93. The device of claim 92, further comprising:
a wavefront sensor that includes:

one or more lenses positioned to receive the returned light;
an array of lenses that is distinct and separate from the one or more lenses, the array of lenses being positioned to focus at least a portion of the light received by the one or more lenses; and
a second image sensor positioned to receive the light focused by the array of lenses.

Clause 94. The device of claim 93, further comprising:
a beam steerer positioned to allow at least a portion of the returned light to propagate toward the first image sensor and allow at least a portion of the returned light to propagate toward the second image sensor.

Clause 95. The device of claim 94, wherein:
the beam steerer includes a partial reflector.

Clause 96. The device of claim 94 or 95, wherein:
the beam steerer includes a movable reflector.

Clause 97. The device of any of claims 93-96, further comprising:
one or more communication interfaces for sending information based on images obtained by the first image sensor and the second image sensor to an electronic device that is located remotely from the device for fabrication of a contact lens.

Clause 98. A method, comprising:

> providing light to an eye wearing a contact lens with one or more marks;
> receiving at least a portion of the light returned from the eye; and
> imaging the received light to form an image with respective shadows of the one or more marks.

Clause 99. The method of claim 98, further comprising:
determining a position and a rotation of the contact lens on the eye based on the image with respective shadows of the one or more marks.

The foregoing description, for purpose of explanation, has been described with reference to specific embodiments. However, the illustrative discussions above are not intended to be exhaustive or to limit the scope of claims to the precise forms disclosed. Many modifications and variations are possible in view of the above teachings. For example, the contact lens with a scribed mark may be a multifocal lens. In another example, the methods described above may be used for designing and making lenses for spectacles (e.g., eyeglasses). The embodiments were chosen and described in order to best explain the principles of the various described embodiments and their practical applications, to thereby enable others skilled in the art to best utilize the invention and the various described embodiments with various modifications as are suited to the particular use contemplated.

**Claims**

1. A contact lens with a plurality of marks for indicating a position of the contact lens and a rotation of the contact lens while the contact lens is positioned on an eye of a user, wherein the plurality of marks includes at least one mark that is scribed on the contact lens.

2. The contact lens of claim 1, wherein the plurality of marks includes a first set of one or more marks for indicating the position of the contact lens.

3. The contact lens of claim 2, wherein the first set of one or more marks includes a mark positioned adjacent to a center of the contact lens.

4. The contact lens of any of claims 1-3, wherein the plurality of marks includes a second set of one or more marks for indicating the rotation of the contact lens.

5. The contact lens of claim 4, wherein the second set of one or more marks includes a mark positioned at least partially within an area corresponding to a pupil of the eye.

6. The contact lens of any of claims 1-5, wherein the scribed mark is located on an anterior surface of the contact lens.

7. The contact lens of claim 6, wherein the anterior surface of the contact lens includes a region having a convex surface.

8. The contact lens of any of claims 1-7, wherein the plurality of marks includes at least one mark that is positioned on the contact lens without using a dye.

9. A method of making the contact lens of any of claims 1-8, the method comprising:

> obtaining a first contact lens; and
> forming one or more indentations on the first contact lens for indicating a position of the contact lens and a rotation of the contact lens.

10. The method of claim 9, wherein forming the one or more indentations on the first contact lens includes scribing the one or more indentations on the first contact lens.

11. The method of claim 9 or 10, wherein obtaining the first contact lens includes:

> obtaining a substrate; and
> removing one or more portions of the substrate to obtain the first contact lens.

12. The method of any of claims 9-11, wherein:
the one or more indentations are formed on an anterior surface of the first contact lens.

13. A device, comprising:

> a light source for providing light to an eye so that at least a portion of the light is returned to the device;
> a first image sensor positioned to image a pupil region of the eye for capturing a shadow of one or more marks on a contact lens positioned on the eye;

14. The device of claim 13, further comprising:
a wavefront sensor that includes:

> one or more lenses positioned to receive the returned light;
> an array of lenses that is distinct and separate from the one or more lenses, the array of lenses being positioned to focus at least a portion of the light received by the one or more lenses; and a second image sensor positioned to receive the light focused by the array of lenses.

15. A method, comprising:

providing light to an eye wearing a contact lens with one or more marks;
receiving at least a portion of the light returned from the eye; and
imaging the received light to form an image with respective shadows of the one or more marks.

**Figure 1A**

**Figure 1B**

**Figure 1C**

**Figure 1D**

**Figure 1E**

**Figure 1F**

**Figure 1G**

**Figure 1H**

— 104

254 —
User Interface

202 —
Processors

— 208

204 —
Communication Interface

252 —
Peripherals Controller

Memory 206 —

| | |
|---|---|
| Operating System | — 210 |
| Communications Module | — 212 |
| Web Browser | — 214 |
|    Position Characterization Web Application | — 216 |
| Vision Characterization Application | — 218 |
|    Reference Marking Identification Module | — 220 |
|       Position Reference Marking Identification Module | — 222 |
|       Angular Reference Marking Identification Module | — 224 |
|       Illumination Marking Identification Module | — 226 |
|    Reference Point Identification Module | — 228 |
|    Wavefront Analysis Module | — 230 |
|    Lens Surface Profile Determination Module | — 232 |
| Measurement Device Module | — 234 |
| User Input Module | — 236 |
|      ⋮ | |
| Database(s) | — 238 |

# Figure 2

Figure 3C

Figure 3B

Figure 3A

Figure 3D

**Figure 3E**

**Figure 4A**

**Figure 4B**

**Figure 5A**

**Figure 5B**

**Figure 6**

**Figure 7A**

**Figure 7B**

**Figure 8A**

**Figure 8B**

**Figure 8C**

**Figure 8D**

x-axis tilt

TOP view

E

L1

E

E

front

y

x

C1

C2

$m_p$

y

x

y-axis tilt    L1

SIDE view

E

E

E

front

y

x

C1

C2

$m_p$

y

x

## Figure 9

$$sag = R - \sqrt{R^2 - r^2}$$

$$\alpha = \sin^{-1}\left(\frac{x_1}{sag_2 - sag_1}\right)$$

$$x_2 = sag_1 \sin \alpha = \left(\frac{sag_1\, x_1}{sag_2 - sag_1}\right)$$

View Angle

L0

L2

C2

$r_1$

$sag_1$ $\alpha$

$x_2$

$x_1$

$sag_2 - sag_1$ $\alpha$

C1

$r_2$

$sag_2$

L1

$R$ = radius of curvature

**Figure 10**

EP 3 936 926 A1

Lens center difference (Diff X: -0.15 mm; Diff Y: -0.35 mm; Difference: 0.38 mm)

Lens Tilt Angle: 7.20 deg
Center Point Difference: 0.15 mm

C1

C2

VA

L0

Figure 11

**Figure 12A**

**Figure 12B**

**Figure 12C**

**Figure 13A**

**Figure 13B**

**Figure 14A**

**Figure 14B**

y
z

170

154

122  110  126

130

128

156

160

132  140

1403

**Figure 14C**

y
z

170

154

122  110

126

130

128

156

160

132  140

1403

**Figure 14D**

**Figure 15**

Europäisches
Patentamt

European
Patent Office

Office européen
des brevets

## EUROPEAN SEARCH REPORT

Application Number

EP 21 18 3719

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/015290 A2 (TECHNOVISION GMBH GES FUER DIE [DE]; POLLAND HANS-JOACHIM [DE] ET AL.) 17 February 2005 (2005-02-17) * page 8, line 8 - page 29, line 7; figures 1-34 * | 1-15 | INV. G02C7/02 G02C7/04 |
| X | WO 2011/014510 A1 (JOHNSON & JOHNSON VISION CARE [US]; CHEHAB KHALED A [US] ET AL.) 3 February 2011 (2011-02-03) | 1,2,4,6, 7,9-12 | |
| Y | * page 2, line 13 - page 10, line 6; figures 1-7 * | 1-15 | |
| X | GB 2 458 495 A (VEEN DAVID RICHARD HENDRIK [GB]) 23 September 2009 (2009-09-23) | 1,2,4,9 | |
| Y | * page 2, line 15 - line 19; figure 7 * | 1-15 | |
| X | EP 2 031 432 A2 (MENICON CO LTD [JP]) 4 March 2009 (2009-03-04) | 1,9-12 | |
| Y | * line 146, paragraph 77; figures 1-13B; examples 1-3 * | 1-15 | |
| X | US 6 086 202 A (CHATEAU NICOLAS [FR] ET AL) 11 July 2000 (2000-07-11) | 1,2,4,9 | TECHNICAL FIELDS SEARCHED (IPC) |
| Y | * column 2, line 56 - column 4, line 53; figures 4-7 * | 1-15 | G02C |
| X | WO 2008/081599 A1 (MENICON CO LTD [JP]; ONISHI TORU [JP] ET AL.) 10 July 2008 (2008-07-10) | 1,9-12 | |
| Y | * paragraph [0043] - paragraph [0134]; figures 1-17 * | 1-15 | |
| X | US 6 042 230 A (NEADLE SUSAN [US] ET AL) 28 March 2000 (2000-03-28) | 1,9 | |
| Y | * column 1, line 34 - column 4, line 5; figures 1-2B * | 1-15 | |

The present search report has been drawn up for all claims

| Place of search | Date of completion of the search | Examiner |
|---|---|---|
| Munich | 24 November 2021 | Bratfisch, Knut |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.                    EP 21 18 3719

This annex lists the patent family members relating to the patent documents cited in the above-mentioned European search report.
The members are as contained in the European Patent Office EDP file on
The European Patent Office is in no way liable for these particulars which are merely given for the purpose of information.

24-11-2021

| Patent document cited in search report | Publication date | Patent family member(s) | | Publication date |
|---|---|---|---|---|
| WO 2005015290 A2 | 17-02-2005 | AU | 2004263956 A1 | 17-02-2005 |
| | | CA | 2533068 A1 | 17-02-2005 |
| | | CN | 1886690 A | 27-12-2006 |
| | | DE | 10333794 A1 | 03-03-2005 |
| | | EP | 1654579 A2 | 10-05-2006 |
| | | ES | 2397518 T3 | 07-03-2013 |
| | | JP | 4691030 B2 | 01-06-2011 |
| | | JP | 2006528787 A | 21-12-2006 |
| | | KR | 20060061799 A | 08-06-2006 |
| | | SG | 137867 A1 | 28-12-2007 |
| | | US | 2007273828 A1 | 29-11-2007 |
| | | WO | 2005015290 A2 | 17-02-2005 |
| WO 2011014510 A1 | 03-02-2011 | AR | 077613 A1 | 07-09-2011 |
| | | AR | 104544 A2 | 26-07-2017 |
| | | AU | 2010276507 A1 | 16-02-2012 |
| | | BR | 112012002220 A2 | 31-05-2016 |
| | | CA | 2769345 A1 | 03-02-2011 |
| | | CN | 102549479 A | 04-07-2012 |
| | | CN | 105137614 A | 09-12-2015 |
| | | CN | 106226918 A | 14-12-2016 |
| | | EP | 2460047 A1 | 06-06-2012 |
| | | JP | 5872469 B2 | 01-03-2016 |
| | | JP | 2013501252 A | 10-01-2013 |
| | | KR | 20120051021 A | 21-05-2012 |
| | | RU | 2012107342 A | 10-09-2013 |
| | | SG | 177770 A1 | 29-03-2012 |
| | | TW | 201131235 A | 16-09-2011 |
| | | US | 2011025979 A1 | 03-02-2011 |
| | | WO | 2011014510 A1 | 03-02-2011 |
| GB 2458495 A | 23-09-2009 | NONE | | |
| EP 2031432 A2 | 04-03-2009 | EP | 2031432 A2 | 04-03-2009 |
| | | JP | 4820348 B2 | 24-11-2011 |
| | | JP | 2009058828 A | 19-03-2009 |
| | | US | 2009059166 A1 | 05-03-2009 |
| US 6086202 A | 11-07-2000 | CA | 2266046 A1 | 07-10-1999 |
| | | EP | 0949530 A1 | 13-10-1999 |
| | | FR | 2777093 A1 | 08-10-1999 |
| | | US | 6086202 A | 11-07-2000 |
| WO 2008081599 A1 | 10-07-2008 | EP | 2101211 A1 | 16-09-2009 |
| | | JP | WO2008081599 A1 | 30-04-2010 |
| | | WO | 2008081599 A1 | 10-07-2008 |

EPO FORM P0459

For more details about this annex : see Official Journal of the European Patent Office, No. 12/82

**ANNEX TO THE EUROPEAN SEARCH REPORT
ON EUROPEAN PATENT APPLICATION NO.**

EP 21 18 3719

This annex lists the patent family members relating to the patent documents cited in the above-mentioned European search report.
The members are as contained in the European Patent Office EDP file on
The European Patent Office is in no way liable for these particulars which are merely given for the purpose of information.

24-11-2021

| Patent document cited in search report | Publication date | Patent family member(s) | | Publication date |
|---|---|---|---|---|
| US 6042230 A | 28-03-2000 | AR | 021639 A1 | 31-07-2002 |
| | | AU | 758002 B2 | 13-03-2003 |
| | | BR | 9907388 A | 03-10-2000 |
| | | CA | 2291969 A1 | 14-06-2000 |
| | | CN | 1260508 A | 19-07-2000 |
| | | EP | 1014152 A1 | 28-06-2000 |
| | | JP | 4443700 B2 | 31-03-2010 |
| | | JP | 2000199875 A | 18-07-2000 |
| | | KR | 20000048029 A | 25-07-2000 |
| | | SG | 87857 A1 | 16-04-2002 |
| | | TW | 468800 U | 11-12-2001 |
| | | US | 6042230 A | 28-03-2000 |

EPO FORM P0459

For more details about this annex : see Official Journal of the European Patent Office, No. 12/82

page 2 of 2